# EUROPEAN PATENT APPLICATION

(11) **EP 1 143 004 A2**
(43) Date of publication of application: **10.10.2001**
(21) Application number: 01102789.3
(22) Date of filing: 04.06.1991
(51) Int. Cl.: C12N 15/32, C07K 14/325, C12N 1/20, A01N 63/00, C12N 15/82, C12R 1/07

(54) **Bacillus thuringiensis microbes active against nematodes, and genes encoding novel nematode-active toxins cloned from bacillus thuringiensis isolates**

(30) Priority: 11.06.1990 US 535810; 24.07.1990 US 557246; 27.07.1990 US 558738; 10.08.1990 US 565544; 14.03.1991 US 669126; 27.03.1991 US 675772; 03.05.1991 US 693018
(62) Divisional of application: 91305047.2
(71) Applicant: Mycogen Corporation, San Diego, CA 92121 (US)
(72) Inventor: Narva, Kenneth E., Carlsbad, CA 92009 (US); Payne, Jewel M., Davis, CA 95616 (US); Schwab, George E., Encinitas, CA 92024 (US); Hickle, Leslie Ann, Chula Vista, CA 91010 (US); Galasan, Theresa, San Diego, California 92130 (US); Sick, August J., Carlsbad California 92008 (US)
(74) Representative: Perry, Robert Edward

(57) **Abstract**

The subject invention concerns novel isolates of, and genes from, *Bacillus thuringiensis* (B.t.) which express toxins having nematicidal activity. Also described are methods for controlling nematodes and flukes.

## Description

### Background of the Invention

Regular use of chemicals to control unwanted organisms can select for drug resistant strains. This has occurred in many species of economically important insects and has also occurred in nematodes of sheep, goats, and horses. The development of drug resistance necessitates a continuing search for new control agents having different modes of action.

In recent times, the accepted methodology for control of nematodes has centered around the drug benzimidazole and its congeners. The use of these drugs on a wide scale has led to many instances of resistance among nematode populations (Prichard, R.K. et al. [1980] "The problem of anthelmintic resistance in nematodes," Austr. Vet. J. 56:239-251; Coles, G.C. [1986] "Anthelmintic resistance in sheep," In Veterinary Clinics of North America: Food Animal Practice Vol 2:423-432 [Herd, R.P., eds.] W.B. Saunders, New York). There are more than 100,000 described species of nematodes.

The bacterium Bacillus thuringiensis (B.t.) produces a δ-endotoxin polypeptide that has been shown to have activity against a rapidly growing number of insect species. The earlier observations of toxicity only against lepidopteran insects have been expanded with descriptions of B.t. isolates with toxicity to dipteran and coleopteran insects. These toxins are deposited as crystalline inclusions within the organism. Many strains of B.t. produce crystalline inclusions with no demonstrated toxicity to any insect tested.

A small number of research articles have been published about the effects of delta endotoxins from B. thuringiensis species on the viability of nematode eggs. Bottjer, Bone and Gill (Experimental Parasitology 60:239-244, 1985) have reported that B.t. kurstaki and B.t. israelensis were toxic in vitro to eggs of the nematode Trichostrongylus colubriformis. In addition, 28 other B.t. strains were tested with widely variable toxicities. The most potent had LD₅₀ values in the nanogram range. Ignoffo and Dropkin (Ignoffo, C.M. and Dropkin, V.H. [1977] J. Kans. Entomol. Soc. 50:394-398) have reported that the thermostable toxin from Bacillus thuringiensis (beta exotoxin) was active against a free-living nematode, Panagrellus redivivus (Goodey); a plant-parasitic nematode, Meloidogyne incognita (Chitwood); and a fungus-feeding nematode, Aphelenchus avena (Bastien). Beta exotoxin is a generalized cytotoxic agent with little or no specificity. Also, H. Ciordia and W.E. Bizzell (Jour. of Parasitology 47:41 [abstract] 1961) gave a preliminary report on the effects of B. thuringiensis on some cattle nematodes.

Flukes belong to subclass Digenea, class Trematoda in the phylum Platyhelminthes. These digeneans all have an intermediate host and are found exclusively in vertebrates, including man. Families which have members of considerable veterinary importance are Fasciolidae, Dicrocoeliidae, Paramphistomatidae, and Schistosomatidae. The adult trematode flukes occur primarily in the bile ducts, alimentary tract, and vascular system. Depending on the predilection site, the eggs pass out of the final host, usually in faeces or urine, and the larval stages develop in a molluscan intermediate host.

Several clinical syndromes may be associated with liver fluke (Fasciola sp.) infection, depending on the numbers and stage of development of the parasite and on the presence or absence of certain bacteria (Clostridium novyi). Acute fluke disease occurs during invasion of the liver by recently ingested metacercariae. Sheep can die very quickly due to focal liver necrosis and extensive subcutaneous hemorrhage.

Anthelmintic medication in the U.S. currently consists primarily of albendazole, which is available in only a few states where Fasciola hepatica poses a serious problem. Special dispensation is required to treat sheep with albendazole elsewhere in the U.S. Other effective flukicides―diamphenethide, nitroxynil, oxyclozanide, rafoxanide, and triclabendazole―are not available in the U.S.

At the present time there is a need to have more effective means to control the many nematodes and flukes that cause considerable damage to susceptible hosts. Advantageously, such effective means would employ biological agents.

### Brief Summary of the Invention

The subject invention concerns isolates, genes, and gene fragments of Bacillus thuringiensis that produce biologically active toxins. These toxins have been shown to be active against nematodes. Specifically exemplified is activity against Caenorhabditis elegance, Pratylenchus spp., and Panagrellus redivivus. Panagrellus redivivus, which is also called the beer-mat nematode, is a common free-living nematode that is relatively easy to maintain in the laboratory. It is often used as an indicator (model) of nematode activity. The toxins of the subject invention can also be uesd to control flukes, including the liver fluke Fasciola hepatica.

The novel B.t. isolates of the subject invention are B.t. strain PS80JJ1, B.t. strain PS158D5, B.t. strain PS167P, B.t. strain PS169E, B.t. strain PS177F1, B.t. strain PS177G, B.t. strain PS204G4, and B.t. strain PS204G6.

The B.t. isolates used according to the subject invention can be grown and the δ-endotoxin that is produced recovered by standard procedures and as further described herein. The recovered toxin, or the B.t. isolates themselves, can be formulated using standard procedures associated with the use of nematicidal or flukicidal products.

The subject invention further concerns genes or gene fragments cloned from Bacillus thuringiensis isolates designated B.t. PS17, B.t. PS33F2, PS63B, PS52A1, and PS69D1. The subject invention concerns four genes cloned from the Bacillus thuringiensis isolate designated B.t. PS17. These genes have been designated PS17a, PS17b, PS17d and PS17e. The genes from PS17, as well as unique genes from each of the B.t. isolates designated B.t. PS33F2, B.t. PS63B, B.t. PS52A1, B.t. PS69D1, encode Bacillus thuringiensis δ-endotoxins which have nematicidal activity. The genes, or gene fragments, of the subject invention can be transferred to suitable hosts via a recombinant DNA vector.

### Brief Description of the Sequences

**Sequence 1** discloses the DNA of PS17a.

**Sequence 2** discloses the amino acid sequence of the toxin encoded by PS17a.

**Sequence 3** discloses the DNA of PS17b.

**Sequence 4** discloses the amino acid sequence of the toxin encoded by PS17b.

**Sequence 5** is the nucleotide sequence of a gene from PS33F2.

**Sequence 6** is the amino acid sequence of the protein expressed by the gene from PS33F2.

**Sequence 7** is the nucleotide sequence of a gene from PS52A1.

**Sequence 8** is the amino acid sequence of the protein expressed by the gene from PS52A1.

**Sequence 9** is the nucleotide sequence of a gene from PS69D1.

**Sequence 10** is the amino acid sequence of the protein expressed by the gene from PS69D1.

### Detailed Disclosure of the Invention

Among the B.t. isolates which can be used according to the subject invention are B.t. PS17, B.t. PS33F2, B.t. PS52A1, B.t. PS63B, B.t. PS69D1, B.t. PS80JJ1, B.t. PS158D5, B.t. PS167P, B.t. PS169E, B.t. PS177F1, B.t. PS177G, B.t. PS204G4, and B.t. PS204G6.

Novel toxin genes or gene fragments of the subject invention can be obtained from nematode-active B. thuringiensis (B.t.) isolates designated PS17, PS33F2, PS63B, PS52A1, and PS69D1. Genes coding for nematacidal toxins can also be obtained from B.t. PS80JJ1, B.t. PS158D5, B.t. PS167P, B.t. PS169E, B.t. PS177F1, B.t. PS177G, B.t. PS204G4, and B.t. PS204G6. Subcultures of the Bacillus thuringiensis isolates and E. coli host harboring the toxin genes of the invention were deposited in the permanent collection of the Northern Research Laboratory, U.S. Department of Agriculture, Peoria, Illinois, USA. The accession numbers are as follows:

| Culture | Repository No. | Deposit Date |
|---|---|---|
| B.t. isolate PS17 | NRRL B-18243 | July 28, 1987 |
| B.t. isolate PS33F2 | NRRL B-18244 | July 28, 1987 |
| B.t. isolate PS63B | NRRL B-18246 | July 28, 1987 |
| B.t. isolate PS52A1 | NRRL B-18245 | July 28, 1987 |
| B.t. isolate PS69D1 | NRRL B-18247 | July 28, 1987 |
| E. coli NM522(pMYC1627) | NRRL B-18651 | May 11, 1990 |
| E. coli NM522(pMYC1628) | NRRL B-18652 | May 11, 1990 |
| B.t. PS80JJ1 | NRRL B-18679 | July 17, 1990 |
| B.t. PS158D5 | NRRL B-18680 | July 17, 1990 |
| B.t. PS167P | NRRL B-18681 | July 17, 1990 |
| B.t. PS169E | NRRL B-18682 | July 17, 1990 |
| B.t. PS177F1 | NRRL B-18683 | July 17, 1990 |
| B.t. PS177G | NRRL B-18684 | July 17, 1990 |
| B.t. PS204G4 | NRRL B-18685 | July 17, 1990 |
| B.t. PS204G6 | NRRL B-18686 | July 17, 1990 |
| E. coli NM522(pMYC 2321) | NRRL B-18770 | February 14, 1991 |
| E. coli NM522(pMYC 2316) | NRRL B-18785 | March 15, 1991 |
| E. coli NM522(pMYC 2317) | NRRL B-18816 | April 24, 1991 |

The toxin genes used according to the subject invention can be obtained, for example, from the B. thuringiensis isolate designated PS17. As shown above, a subculture of B.t. PS17 harboring toxin genes of the invention has been deposited. Similar subcultures of the B.t. isolates B.t. PS33F2, B.t. PS63B, B.t. PS52A1, B.t. PS69D1, and E. coli hosts harboring toxin genes of the invention have also been deposited.

The subject cultures have been deposited under conditions that assure that access to the cultures will be available during the pendency of this patent application to one determined by the Commissioner of Patents and Trademarks to be entitled thereto under 37 CFR 1.14 and 35 USC 122. The deposits are available as required by foreign patent laws in countries wherein counterparts of the subject application, or its progeny, are filed. However, it should be understood that the availability of a deposit does not constitute a license to practice the subject invention in derogation of patent rights granted by governmental action.

Further, the subject culture deposits will be stored and made available to the public in accord with the provisions of the Budapest Treaty for the Deposit of Microorganisms, i.e., they will be stored with all the care necessary to keep them viable and uncontaminated for a period of at least five years after the most recent request for the furnishing of a sample of the deposit, and in any case, for a period of at least 30 (thirty) years after the date of deposit or for the enforceable life of any patent which may issue disclosing the cultures. The depositor acknowledges the duty to replace the deposits should the depository be unable to furnish a sample when requested, due to the condition of the deposit(s). All restrictions on the availability to the public of the subject culture deposits will be irrevocably removed upon the granting of a patent disclosing them.

The novel B.t. genes of the invention encode toxins which show activity against tested nematodes. The group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes wide-spread and often times serious infection in various species of animals. The most common genera of nematodes infecting the animals referred to above are Haemonchus, Trichostrongylus, Ostertagia, Nematodirus, Cooneria, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris, Caenorhabditis and Parascaris. Certain of these, such as Nematodirus, Cooperia, and Oesophagostomum, attack primarily the intestinal tract, while others, such as Dictyocaulus are found in the lungs. Still other parasites may be located in other tissues and organs of the body.

The toxins encoded by the novel B.t. genes of the subject invention are useful as nematocides for the control of soil nematodes and plant parasites selected from the genera Bursaphalenchus, Criconemella, Ditylenchus, Globodera, Helicotylenchus, Heterodera, Melodiogyne, Pratylenchus, Radolpholus, Rotelynchus, or Tylenchus.

The methods and compositions of the subject invention can also be used to control liver flukes, which can parasitize vertebrates. Specifically, the invention can be used to control flukes in humans, livestock, domestic pets, and other animals. As used herein, the term "livestock" can include, for example, sheep, cattle, pigs, and goats. The methods and compositions of the subject invention may be used to control immature and adult flukes. The methods of control include, but are not limited to, pasture treatment (for vertebrate and intermediate hosts); liposomes or other carriers for delivering the toxins to the liver or bile ducts; and treatment of free-living forms of the flukes in the gastrointestinal tracts of vertebrate hosts. The flukicidal B.t. toxins described herein may be used alone, or in rotation or combination with other flukicides such as, for example, albendazole.

As described herein, B.t. isolates of the invention have activity against liver flukes. It is expected that these isolates would be active against other flukes as disclosed herein.

The B.t. toxins of the invention can be administered orally in a unit dosage form such as a capsule, bolus or tablet, or as a liquid drench when used as an anthelmintic in mammals and in the soil to control plant nematodes. The drench is normally a solution, suspension or dispersion of the active ingredient, usually in water, together with a suspending agent such as bentonite and a wetting agent or like excipient. Generally, the drenches also contain an antifoaming agent. Drench formulations generally contain from about 0.001 to 0.5% by weight of the active compound. Preferred drench formulations may contain from 0.01 to 0.1% by weight, the capsules and boluses comprise the active ingredient admixed with a carrier vehicle such as starch, talc, nagnesium stearate, or dicalcium phosphate.

Where it is desired to administer the toxin compounds in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with respect to their total weight and content of the active agent, depending upon the factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or, optionally, fed separately. Alternatively, the toxin compounds may be administered to animals parenterally, for example, by intraruminal, intramuscular, intratracheal, or subcutaneous injection, in which event the active ingredient is dissolved or dispersed in a liquid carrier vehicle. For parenteral administration, the active material is suitably admixed with an acceptable vehicle, preferably of the vegetable oil variety, such as peanut oil, cotton seed oil and the like. Other parenteral vehicles, such as organic preparations using solketal, glycerol, formal and aqueous parenteral formulations, are also used. The active compound or compounds are dissolved or suspended in the parenteral formulation for administration; such formulations generally contain from 0.005 to 5% by weight of the active compound.

When the toxins are administered as a component of the feed of the animals, or dissolved or suspended in the drinking water, compositions are provided in which the active compound or compounds are intimately dispersed in an inert carrier or diluent. By inert carrier is meant one that will not react with the active agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like.

In addition to having nematicidal and flukicidal activity within the digestive tract of mammals, spores from the B.t. isolates will pass through the animals' digestive tract, germinate and multiply in the feces, and thereby provide additional control of larva which hatch and multiply therein.

The gene(s) from the novel B.t. isolates of the subject invention can be introduced into microbes capable of occupying, surviving in, and proliferating in the phytosphere of plants according to the procedure of European Patent Application 0 200 344. Upon ingestion of such a plant by an animal hosting a nematode or fluke, the toxin becomes available in the animal host to control the nematode or fluke infestation.

The toxin genes or gene fragments of the subject invention can be introduced into a wide variety of microbial hosts. Expression of the toxin gene results, directly or indirectly, in the intracellular production and maintenance of the nematicide. With suitable hosts, e.g., Pseudomonas, the microbes can be applied to the situs of nematodes where they will proliferate and be ingested by the nematodes. The result is a control of the nematodes. Alternatively, the microbe hosting the toxin gene can be treated under conditions that prolong the activity of the toxin produced in the cell. The treated cell then can be applied to the environment of target pest(s). The resulting product retains the toxicity of the B.t. toxin.

A wide variety of ways are available for introducing the B.t. gene expressing the toxin into the microorganism host under conditions which allow for stable maintenance and expression of the gene. One can provide for DNA constructs which include the transcriptional and translational regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger, particularly by using an active promoter, as well as by employing sequences, which enhance the stability of the messenger RNA. The initiation and translational termination region will involve stop codon(s), a terminator region, and optionally, a polyadenylation signal.

In the direction of transcription, namely in the 5' to 3' direction of the coding or sense sequence, the construct will involve the transcriptional regulatory region, if any, and the promoter, where the regulatory region may be either 5' or 3' of the promoter, the ribosomal binding site, the initiation codon, the structural gene having an open reading frame in phase with the initiation codon, the stop codon(s), the polyadenylation signal sequence, if any, and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototropy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may effectively compete with the wild-type microorganisms and stably occupy a niche in the environment.

Where no functional replication system is present, the construct will also include a sequence of at least 50 basepairs (bp), preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the trp gene, lac gene, gal gene, the lambda left and right promoters, the Tac promoter, the naturally-occurring promoters associated with the toxin gene, where functional in the host. See for example, U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome, an episomal element normally present in the host or a different host, or a replication system from a virus which is stable in the host. A large number of plasmids are available, such as pBR322, pACYC184, RSF1010, pRO1614, and the like. See for example, Olson et al., (1982) J. Bacteriol. 150:6069, and Bagdasarian et al., (1981) Gene 16:237, and U.S. Patent Nos. 4,356,270, 4,362,817, and 4,371,625.

The B.t. gene can be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the host genome.

The transformants can be isolated in accordance with conventional ways, usually employing a selection technique, which allows for selection of the desired organism as against unmodified organisms or transferring organisms, when present. The transformants then can be tested for nematicidal or flukicidal activity.

Where the B.t. toxin gene or gene fragment is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the nematicide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, e.g., genera Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc, and Alcaligenes; fungi, particularly yeast, e.g., genera Saccharomyces, Cryptococcus, Kluyveromyces, Sporobolomyces, Rhodotorula, and Aureobasidium. Of particular interest are such phytosphere bacterial species as Pseudomonas syringae. Pseudomonas fluorescens, Serratia marcescens, Acetobacter xylinum, Agrobacterium tumefaciens, Rhodopseudomonas spheroides, Xanthomonas campestris, Rhizobium melioti, Alcaligenes entrophus, and Azotobacter vinlandii; and phytosphere yeast species such as Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae, and Aureobasidium pollulans. Of particular interest are the pigmented microorganisms.

Suitable host cells, where the nematicide- or flukicide-containing cells will be treated to prolong the activity of the toxin in the cell when the then treated cell is applied to the environment of target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and - positive, include Enterobacteriaceae, such as Escherichia, Erwinia, Shigella, Salmonella, and Proteus; Bacillaceae; Rhizobiceae, such as Rhizobium; Spirillaceae, such as photobacterium, Zymomonas, Serratia, Aeromonas, Vibrio, Desulfovibrio, Spirillum; Lactobacillaceae; Pseudomonadaceae, such as Pseudomonas and Acetobacter; Azotobacteraceae and Nitrobacteraceae. Among eukaryotes are fungi, such as Phycomycetes and Ascomycetes, which includes yeast, such as Saccharomyces and Schizosaccharomyces; and Basidiomycetes yeast, such as Rhodotorula, Aureobasidium, Sporobolomyces, and the like.

Characteristics of particular interest in selecting a host cell for purposes of production include ease of introducing the B.t. gene into the host, availability of expression systems, efficiency of expression, stability of the nematicide or flukicide in the host, and the presence of auxiliary genetic capabilities. Characteristics of interest for use as a nematicide or flukicide microcapsule include protective qualities for the toxin, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as Rhodotorula sp., Aureobasidium sp., Saccharomyces sp., and Sporobolomyces sp.; phylloplane organisms such as Pseudomonas sp., Erwinia sp. and Flavobacterium sp.; or such other organisms as Escherichia, Lactobacillus sp., Bacillus sp., and the like. Specific organisms include Pseudomonas aeruginosa, Pseudomonas fluorescens, Saccharomyces cerevisiae, Bacillus thuringiensis, Escherichia coli, Bacillus subtilis, and the like.

The cell will usually be intact and be substantially in the proliferative form when treated, rather than in a spore form, although in some instances spores may be employed.

Treatment of the microbial cell, e.g., a microbe containing the B.t. toxin gene or gene fragment, can be by chemical or physical means, or by a combination of chemical and/or physical means, so long as the technique does not deleteriously affect the properties of the toxin, nor diminish the cellular capability in protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Bouin's fixative and Helly's fixative (See: Humason, Gretchen L., Animal Tissue Techniques, W.H. Freeman and Company, 1967); or a combination of physical (heat) and chemical agents that preserve and prolong the activity of the toxin produced in the cell when the cell is administered to the host animal. Examples of physical means are short wavelength radiation such as gamma-radiation and X-radiation, freezing, UV irradiation, lyophilization, and the like.

The cells generally will have enhanced structural stability which will enhance resistance to environmental conditions. Where the pesticide is in a proform, the method of inactivation should be selected so as not to inhibit processing of the proform to the mature form of the pesticide by the target pest pathogen. For example, formaldehyde will crosslink proteins and could inhibit processing of the proform of a polypeptide pesticide. The method of inactivation or killing retains at least a substantial portion of the bio-availability or bioactivity of the toxin.

The cellular host containing the B.t. toxin gene or gene fragment may be grown in any convenient nutrient medium, where the DNA construct provides a selective advantage, providing for a selective medium so that all, or substantially all, of the cells retain the B.t. gene. These cells may then be harvested in accordance with conventional ways. Alternatively, the cells can be treated prior to harvesting.

The B.t. cells may be formulated in a variety of ways. They may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The toxin concentration will vary widely depending upon the nature of the particular formulation, particularly whether it is a concentrate or to be used directly. The toxin will be present in at least 1% by weight and may be 100% by weight. The dry formulations will have from about 1-95% by weight of the toxin while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the nematodes or flukes, e.g., plants, soil or water, by spraying, dusting, sprinkling, or the like.

Alternatively, because some plant parasitic nematodes are obligate parasites, genes coding for nematocidal B.t. toxins can be engineered into plant cells to yield nematode-resistant plants. The methodology for engineering plant cells is well established (cf. Nester, E.W., Gordon, M.P., Amasino, R.M. and Yanofsky, M.F., Ann. Rev. Plant Physiol. 35:387-399, 1984).

It is well known in the art that the amino acid sequence of a protein is determined by the nucleotide sequence of the DNA. Because of the redundancy of the genetic code, i.e., more than one coding nucleotide triplet (codon) can be used for most of the amino acids used to make proteins, different nucleotide sequences can code for a particular amino acid. Thus, the genetic code can be depicted as follows:

| | | | |
|---|---|---|---|
| Phenylalanine (Phe) | TTK | Histidine (His) | CAK |
| Leucine (Leu) | XTY | Glutamine (Gln) | CAJ |
| Isoleucine (Ile) | ATM | Asparagine (Asn) | AAK |
| Methionine (Met) | ATG | Lysine (Lys) | AAJ |
| Valine (Val) | GTL | Aspartic acid (Asp) | GAK |
| Serine (Ser) | QRS | Glutamic acid (Glu) | GAJ |
| Proline (Pro) | CCL | Cysteine (Cys) | TGK |
| Threonine (Thr) | ACL | Tryptophan (Trp) | TGG |
| Alanine (Ala) | GCL | Arginine (Arg) | WGZ |
| Tyrosine (Tyr) | TAK | Glycine (Gly) | GGL |
| Termination signal | TAJ | | |

Key: Each 3-letter deoxynucleotide triplet corresponds to a trinucleotide of mRNA, having a 5'-end on the left and a 3'-end on the right. All DNA sequences given herein are those of the strand whose sequence correspond to the mRNA sequence, with thymine substituted for uracil. The letters stand for the purine or pyrimidine bases forming the deoxynucleotide sequence.
A = adenine
G = guanine
C = cytosine
T = thymine
X = T or C if Y is A or G
X = C if Y is C or T
Y = A, G, C or T if X is C
Y = A or G if X is T
W = C or A if Z is A or G
W - C if Z is C or T
Z = A, G, C or T if W is C
Z = A or G if W is A
QR = TC if S is A, G, C or T; alternatively
   QR = AG if S is T or C
J = A or G
K = T or C
L = A, T, C or G
M = A, C or T

The above shows that the novel amino acid sequence of the B.t. toxins can be prepared by equivalent nucleotide sequences encoding the same amino acid sequence of the protein. Accordingly, the subject invention includes such equivalent nucleotide sequences. In addition it has been shown that proteins of identified structure and function may be constructed by changing the amino acid sequence if such changes do not alter the protein secondary structure (Kaiser, E.T. and Kezdy, F.J. [1984] Science 223:249-255). Thus, the subject invention includes mutants of the amino acid sequence depicted herein which do not alter the protein secondary structure, or if the structure is altered, the biological activity is substantially retained. Further, the invention also includes mutants of organisms hosting all or part of a toxin encoding a gene of the invention. Such microbial mutants can be made by techniques well known to persons skilled in the art. For example, UV irradiation can be used to prepare mutants of host organisms. Likewise, such mutants may include asporogenous host cells which also can be prepared by procedures well known in the art.

The various methods employed in the preparation of the plasmids and transformation of host organisms are well known in the art. These procedures are all described in Maniatis, T., Fritsch, E.F., and Sambrook, J. (1982) Molecular Cloning: A Laboratory Manual, Cold Spring Harbor Laboratory, New York. Thus, it is within the skill of those in the genetic engineering art to extract DNA from microbial cells, perform restriction enzyme digestions, electrophorese DNA fragments, tail and anneal plasmid and insert DNA, ligate DNA, transform cells, prepare plasmid DNA, electrophorese proteins, and sequence DNA.

The restriction enzymes disclosed herein can be purchased from Bethesda Research Laboratories, Gaithersburg, MD, or New England Biolabs, Beverly, MA, or Boehringer Mannheim, Indianapolis, IN. The enzymes are used according to the instructions provided by the supplier.

Following are examples which illustrate procedures, including the best mode, for practicing the invention. These examples should not be construed as limiting. All percentages are by weight and all solvent mixture proportions are by volume unless otherwise noted.

### Example 1 ― Culturing B.t. Isolates

A subculture of B.t. isolates can be used to inoculate the following medium, a peptone, glucose, salts medium.

| | |
|---|---|
| Bacto Peptone | 7.5 g/l |
| Glucose | 1.0 g/l |
| KH₂PO₄ | 3.4 g/l |
| K₂HPO₄ | 4.35 g/l |
| Salts Solution | 5.0 ml/l |
| CaCl₂ Solution | 5.0 ml/l |

| Salts Solution (100 ml) | |
|---|---|
| MgSO₄.7H₂O | 2.46 g |
| MnSO₄.H₂O | 0.04 g |
| ZnSO₄.7H₂O | 0.28 g |
| FeSO₄.7H₂O | 0.40 g |

| CaCl₂ Solution (100 ml) | |
|---|---|
| CaCl₂.2H₂O | 3.66 g |
| pH 7.2 | |

The salts solution and CaCl₂ solution are filter-sterilized and added to the autoclaved and cooked broth at the time of inoculation. Flasks are incubated at 30°C on a rotary shaker at 200 rpm for 64 hr.

The above procedure can be readily scaled up to large fermentors by procedures well known in the art.

The B.t. spores and crystals, obtained in the above fermentation, can be isolated by procedures well known in the art. A frequently-used procedure is to subject the harvested fermentation broth to separation techniques, e.g., centrifugation.

### Example 2 ― Purification and Amino Acid Sequencing

A Bacillus thuringiensis (B.t.) isolate which can be used as a source of toxin protein of the subject invention can be, for example, B.t. PS17, B.t. PS5A1, B.t. PS33F2, B.t. PS63B, B.t. PS69D1, B.t. PS80JJ1, B.t. PS158D5, B.t. PS167P, B.t. PS169E, B.t. PS177F1, B.t. PS177G, B.t. PS204G4, and B.t. PS204G6. The isolate can be cultured as described in Example 1 or by using other standard media and fermentation techniques well known in the art. The toxin protein inclusions can be harvested by standard sedimentation centrifugation. The recovered protein inclusions can be partially purified by sodium bromide (28-38%) isopycnic gradient centrifugation (Pfannenstiel, M.A., E.J. Ross, V.C. Kramer, and K.W. Nickerson [1984] FEMS Microbiol. Lett. 21:39). Thereafter the individual toxin proteins can be resolved by solubilizing the crystalline protein complex in an alkali buffer and fractionating the individual proteins by DEAE-sepharose CL-6B (Sigma Chem. Co., St. Louis, MO) chromatography by step-wise increments of increasing concentrations of an NaCl-containing buffer (Reichenberg, D., in Ion Exchangers in Organic and Biochemistry [C. Calmon and T.R.E. Kressman, eds.], Interscience, New York, 1957). Fractions containing protein toxic for the nematode Caenorhabditis elegans (CE), can be bound to PVDF membrane (Millipore, Bedford, MA) by western blotting techniques (Towbin, H., T. Staehelin, and K. Gordon [1979] Proc. Natl. Acad. Sci. USA 76:4350) and the N-terminal amino acids determined by the standard Edman reaction with an automated gas-phase sequenator (Hunkapiller, M.W., R.M. Hewick, W.L. Dreyer, and L.E. Hood [1983] Meth. Enzymol. 91:399). The sequences which have been obtained include:

PS17a | A I L N E L Y P S V P Y N V
PS17b | A I L N E L Y P S V P Y N V
PS33F2 | A T L N E V Y P V N
PS52A1 | M I I D S K T T L P R H S L I N T
PS63B | Q L Q A Q P L I P Y N V L A
PS69D1 | M I L G N G K T L P K H I R L A H I F A T Q N S

In addition, internal amino acid sequence data were derived for PS63B. The toxin protein was partially digested with Staphylococcus aureus V8 protease (Sigma Chem. Co., St. Louis, MO) essentially as described (Cleveland, D.W., S.G. Fischer, M.W. Kirschner, and U.K. Laemmli [1977] J. Biol. Chem. 252:1102). The digested material was blotted onto PVDF membrane and a ca. 28 kDa limit peptide was selected for N-terminal sequencing as described above. The sequence obtained was:

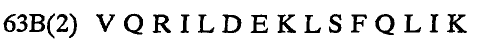

From these sequence data oligonucleotide probes were designed by utilizing a codon frequency table assembled from available sequence data of other B.t. toxin genes. The probes were synthesized on an Applied Biosystems, Inc. DNA synthesis machine.

### Example 3 ― Cloning of Novel Toxin Genes and Transformation into Escherichia coli

### A. B.t. PS17

Total cellular DNA was prepared by growing the cells B.t. PS17 to a low optical density (OD₆₀₀ = 1.0) and recovering the cells by centrifugation. The cells were protoplasted in TES buffer (30 mM Tris-Cl, 10 mM EDTA, 50 mM NaCl, pH = 8.0) containing 20 % sucrose and 50 mg/ml lysozyme. The protoplasts were lysed by addition of SDS to a final concentration of 4%. The cellular material was precipitated overnight at 4°C in 100 mM (final concentration) neutral potassium chloride. The supernate was extracted twice with phenol/chloroform (1:1). The DNA was precipitated with ethanol and purified by isopycnic banding on a cesium chloride-ethidium bromide gradient.

Total cellular DNA from PS17 was digested with EcoRI and separated by electrophoresis on a 0.8% (w/v) Agarose-TAE (50mM Tris-HCl, 20mM NaOAc, 2.5mM EDTA, pH=8.0) buffered gel. A Southern blot of the gel was hybridized with a [³²P] - radiolabeled oligonucleotide probe derived from the N-terminal amino acid sequence of purified 130kDa protein from PS17. The sequence of the oligonucleotide synthesized is (GCAATTTTAAATGAATTATATCC). Results showed that the hybridizing EcoRI fragments of PS17 are 5.0kb, 4.5kb, 2.7kb and 1.8kb in size, presumptively identifying at least four new nematode-active toxin genes, PS17d, PS17b, PS17a and PS17e, respectively.

A library was constructed from PS17 total cellular DNA partially digested with Sau3A and size fractionated by electrophoresis. The 9 to 23kb region of the gel was excised and the DNA was electroeluted and then concentrated using an Elutip™ ion exchange column (Schleicher and Schuel, Keene NH). The isolated Sau3A fragments were ligated into LambdaGEM-11™ (PROMEGA). The packaged phage were plated on KW251 E. coli cells (PROMEGA) at a high titer and screened using the above radiolabeled synthetic oligonucleotide as a nucleic acid hybridization probe. Hybridizing plaques were purified and rescreened at a lower plaque density. Single isolated purified plaques that hybridized with the probe were used to infect KW251 E. coli cells in liquid culture for preparation of phage for DNA isolation. DNA was isolated by standard procedures.

Recovered recombinant phage DNA was digested with EcoRI and separated by electrophoresis on a 0.8% agarose-TAE gel. The gel was Southern blotted and hybridized with the oligonucleotide probe to characterize the toxin genes isolated from the lambda library. Two patterns were present, clones containing the 4.5kb (PS17b) or the 2.7kb (PS17a) EcoRI fragments. Preparative amounts of phage DNA were digested with SalI (to release the inserted DNA from lambda arms) and separated by electrophoresis on a 0.6% agarose-TAE gel. The large fragments, electroeluted and concentrated as described above, were ligated to SalI-digested and dephosphorylated pBClac. The ligation mix was introduced by transformation into NM522 competent E. coli cells and plated on LB agar containing ampicillin, isopropyl-(Beta)-D-thiogalactoside (IPTG) and 5-Bromo-4-Chloro-3-indolyl-(Beta)-D-galactoside(XGAL). White colonies, with putative insertions in the (Beta)-galactosidase gene of pBClac, were subjected to standard rapid plasmid purification procedures to isolate the desired plasmids. The selected plasmid containing the 2.7kb EcoRI fragment was named pMYC1627 and the plasmid containing the 4.5kb EcoRI fragment was called pMYC1628.

The toxin genes were sequenced by the standard Sanger dideoxy chain termination method using the synthetic oligonucleotide probe, disclosed above, and by "walking" with primers made to the sequence of the new toxin genes.

The PS17 toxin genes were subcloned into the shuttle vector pHT3101 (Lereclus, D. et al.[1989] FEMS Microbiol. Lett. 60:211-218) using standard methods for expression in B.t.*.* Briefly, SalI fragments containing the PS17a and PS17b toxin genes were isolated from pMYC1629 and pMYC1627, respectively, by preparative agarose gel electrophoresis, electroelution, and concentrated, as described above. These concentrated fragments were ligated into SalI-cleaved and dephosphorylated pHT3101. The ligation mixtures were used separately to transform frozen, competent E. coli NM522. Plasmids from each respective recombinant E. coli strain were prepared by alkaline lysis and analyzed by agarose gel electrophoresis. The resulting subclones, pMYC2311 and pMYC2309, harbored the PS17a and b toxin genes, respectively. These plasmids were transformed into the acrystalliferous B.t. strain, HD-1 cryB (Aronson, A., Purdue University, West Lafayette, IN), by standard electroporation techniques (Instruction Manual, Biorad, Richmond, CA).

Recombinant B.t. strains HD-1 cryB [pMYC2311] and [pMYC2309] were grown to sporulation and the proteins purified by NaBr gradient centrifugation as described above for the wild-type B.t. proteins.

### B. B.t. PS52A1

Total cellular DNA was prepared from Bacillus thuringiensis PS52A1 cells grown to an optical density, at 600 nm, of 1.0. Cells were pelleted by centrifugation and resuspended in protoplast buffer (20 mg/ml lysozyme in 0.3 M sucrose, 25 mM Tris-Cl [pH 8.0], 25 mM EDTA). After incubation at 37°C for 1 hour, protoplasts were lysed by two cycles of freezing and thawing. Nine volumes of a solution of 0.1 M NaCl, 0.1% SDS, 0.1 M Tris-Cl were added to complete lysis. The cleared lysate was extracted twice with phenol:chloroform (1:1). Nucleic acids were precipitated with two volumes of ethanol and pelleted by centrifugation. The pellet was resuspended in TE and RNase was added to a final concentration of 50 *µ* g/ml. After incubation at 37°C for 1 hour, the solution was extracted once each with phenol:chloroform (1:1) and TE-saturated chloroform. DNA was precipitated from the aqueous phase by the addition of one-tenth volume of 3 M NaOAc and two volumes of ethanol. DNA was pelleted by centrifugation, washed with 70% ethanol, dried, and resuspended in TE.

Restriction fragment length polymorphism (RFLP) analyses were performed by standard hybridization of Southern blots of B.t. PS52A1 DNA with a ³²P-labeled oligonucleotide probe, Probe 52A1-C. The sequence of this probe is: 
5′ ATG ATT ATT GAT TCT AAA ACA ACA TTA CCA AGA CAT TCA/T
TTA ATA/T AAT ACA/T ATA/T AA 3′
 Hybridizing bands included an approximately 3.6 kbp HindIII fragment and an approximately 8.6 kbp EcoRV fragment.

A gene library was constructed from B.t. PS52A1 DNA partially digested with Sau3A. Partial restriction digests were fractionated by agarose gel electrophoresis. DNA fragments 6.6 to 23 kbp in size were excised from the gel, electroeluted from the gel slice, and recovered by ethanol precipitation after purification on an Elutip-D ion exchange column. The Sau3A inserts were ligated into BamHI-digested LambdaGem-11 (Promega). Recombinant phage were packaged and plated on E. coli KW251 cells (Promega). Plaques were screened by hybridization with the radiolabeled Probe 52A1-C. Hybridizing phage were plaque-purified and used to infect liquid cultures of E. coli KW251 cells for isolation of phage DNA by standard procedures (Maniatis et al.). For subcloning, preparative amounts of DNA were digested with EcoRI and SalI, and electrophoresed on an agarose gel. The approximately 3.1 kbp band containing the toxin gene was excised from the gel, electroeluted from the gel slice, and purified by ion exchange chromatography as above. The purified DNA insert was ligated into EcoRI + SalI-digested pHTBlueII (an E. coli/B. thuringiensis shuttle vector comprised of pBluescript S/K [Stratagene] and the replication origin from a resident B.t. plasmid [D. Lereclus et al. (1989) FEMS Microbiology Letters 60:211-218]). The ligation mix was used to transform frozen, competent E. coli NM522 (ATCC 47000). Transformants were plated on LB agar containing ampicillin, isopropyl-(β)-D-thiogalactoside (IPTG), and 5-bromo-chloro-3-indolyl-(*β*)-D-galactoside (XGAL). Plasmids were purified from putative recombinants by alkaline lysis (Maniatis et al.) and analyzed by electrophoresis of EcoRI and SalI digests on agarose gels. The desired plasmid construct, pMYC2321, contains a toxin gene that is novel compared to the maps of other toxin genes encoding nematicidal proteins.

Plasmid pMYC2321 was introduced into an acrystalliferous B.t. strain cryB by electroporation. Expression of an approximately 55-60 kDa crystal protein was verified by SDS-PAGE analysis and nematicidal activity. NaBr-purified crystals were prepared (Pfannenstiel, M.A., et al., supra) for determination of toxicity of the cloned gene product to Pratylenchus scribneri (Plant-Parasitic) and Panagrellus redivivus (Free-Living). The cloned gene product was active against these nematodes.

### C. B.t. PS33F2

Total cellular DNA was prepared from B.t. PS33F2 cells grown to an optical density, at 600nm, of 1.0. Cells were pelleted by centrifugation and resuspended in protoplast buffer (20 mg/ml lysozyme in 0.3 M sucrose, 25 mM Tris-Cl [pH 8.0], 25 mM EDTA). After incubation at 37°C for lh, protoplasts were lysed by the addition of nine volumes of a solution of 0.1 M NaCl, 0.1% SDS, 0.1 M Tris-Cl followed by two cycles of freezing and thawing. The cleared lysate was extracted twice with phenol:chloroform (1:1). Nucleic acids were precipitated with two volumes of ethanol and pelleted by centrifugation. The pellet was resuspended in 10mM Tris-Cl, 1 mM EDTA (TE) and RNase was added to a final concentration of 50µg/ml. After incubation at 37°C for 1h, the solution was extracted once each with phenol:chloroform (1:1) and TE-saturated chloroform. DNA was precipitated from the aqueous phase by the addition of one-tenth volume of 3M NaOAc and two volumes of ethanol. DNA was pelleted by centrifugation, washed with 70% ethanol, dried, and resuspended in TE.

Plasmid DNA was extracted from protoplasts prepared as described above. Protoplasts were lysed by the addition of nine volumns of a solution of 10mM Tris-Cl, 1 mM EDTA, 0.085 N NaOH, 0.1% SDS, pH=8.0. SDS was added to 1% final concentration to complete lysis. One-half volume of 3M KOAc was then added and the cellular material was precipitated overnight at 4°C. After centrifugation, the DNA was precipitated with ethanol and plasmids were purified by isopycnic centrifugation on cesium chloride-ethidium bromide gradients.

RFLP analyses were performed by standard hybridization of Southern blots of PS33F2 plasmid and total cellular DNA with ³²P-labelled oligonucleotide probes designed to the N-terminal amino acid sequence described in Example 2. 
Probe 33F2A: 5′ GCA/T ACA/T TTA AAT GAA GTA/T TAT 3′
Probe 33F2B: 5′ AAT GAA GTA/T TAT CCA/T GTA/T AAT 3′
 Hybridizing bands included an approximately 5.85 kbp EcoRI fragment. Probe 33F2A and a reverse PCR primer were used to amplify a DNA fragment of approximately 1.8 kbp for use as a hybridization probe for cloning the PS33F2 toxin gene. The sequence of the reverse primer was:

5′ GCAAGCGGCCGCTTATGGAATAAATTCAATT
C/T T/G A/G TC T/A A 3′

A gene library was constructed from PS33F2 plasmid DNA digested with EcoRI. Restriction digests were fractionated by agarose gel electrophoresis. DNA fragments 4.3-6.6 kbp were excised from the gel, electroeluted from the gel slice, and recovered by ethanol precipitation after purification on an Elutip-D ion exchange column (Schleicher and Schuel, Keene NH). The EcoRI inserts were ligated into EcoRI-digested pHTBlueII (an E. coli/B. thuringiensis shuttle vector comprised of pBluescript S/K [Stratagene] and the replication origin from a resident B.t. plasmid [D. Lereclus et al. 1989. FEMS Microbial. Lett. 60:211-218]). The ligation mixture was transformed into frozen, competent NM522 cells (ATCC 47000). Transformants were plated on LB agar containing ampicillin, isopropyl - (Beta)-D-thiogalactoside (IPTG), and 5-bromo-4-chloro-3-indolyl-(Beta)-D-galactoside (XGAL). Colonies were screened by hybridization with the radiolabeled PCR amplified probe described above. Plasmids were purified from putative toxin gene clones by alkaline lysis and analyzed by agarose gel electrophoresis of restriction digests. The desired plasmid construct, pMYC2316, contains an approximately 5.85 kbp EcoRI insert; the toxin gene residing on this DNA fragment (33F2a) is novel compared to the DNA sequences of other toxin genes encoding nematicidal proteins.

Plasmid pMYC2316 was introduced into the acrystalliferous (Cry-) B.t. host, HD-1 CryB by electroporation. Expression of an approximately 120-140 kDa crystal protein was verified by SDS-PAGE analysis. Crystals were purified on NaBr gradients (M.A. Pfannenstiel et al. 1984. supra) for determination of toxicity of the cloned gene product to Pratylenchus spp.

### D. B.t. PS69D1

Total cellular DNA was prepared from B.t. PS69D1 as described above for the other isolates. RFLP analyses were performed by standard hybridization of Southern blots of PS69D1 DNA with a 32P-labeled oligonucleotide probe designated as 69D1-D. The sequence of the 69D1-D probe was: 5′ AAA CAT ATT AGA TTA GCA CAT ATT TTT GCA ACA CAA AA 3′ Hybridizing bands included an approximately 2.0 kbp HindIII fragment.

A gene library was constructed from PS69D1 DNA partially digested with Sau3A. Partial restriction digests were fractionated by agarose gel electrophoresis. DNA fragments 6.6 to 23 kbp in size were excised from the gel, electroeluted from the gel slice, and recovered by ethanol precipitation after purification on an Elutip-D ion exchange column. The Sau3A inserts were ligated into BamHI-digested LambdaGem-11 (Promega, Madison, WI). Recombinant phage were packaged and plated on E. coli KW251 cells (Promega, Madison, WI). Plaques were screened by hybridization with the radiolabeled 69D1-D oligonucleotide probe. Hybridizing phage were plaque-purified and used to infect liquid cultures of E. coli KW251 cells for isolation of phage DNA by standard procedures (Maniatis et al. [1982] Molecular Cloning: A Laboratory Manual, Cold Spring Harbor, NY). For subcloning, preparative amounts of DNA were digested with HindIII and electrophoresed on an agarose gel. The approximately, 2.0 kbp band containing the toxin gene was excised from the gel, electroeluted from the gel slice, and purified by ion exchange chromatography as above. The purified DNA insert was ligated into HindIII-digested pHTBlueII (and E. coli/ B.t. shuttle vector comprised of pBluescript S/K (Stratagene, San Diego, CA) and the replication origin from a resident B.t. plasmid (D. Lereclus et al [1989] FEMS Microbiol. Lett. 60:211-218)). The ligation mix was used to transform frozen, competent E. coli NM522 cells (ATCC 47000). Transformants were plated on LB agar containing 5-Bromo-4-Chloro-3-indolyl-(Beta)-D-galactoside (XGAL). Plasmids were purified from putative recombinants by alkaline lysis (Maniatis et al., ibid.) and analyzed by electorphoresis of HindIII digests on agarose gels. The desired plasmid construct, pMYC2317, contains a toxin gene that is novel compared to the maps of other toxin genes encoding insecticidal proteins.

### E. B.t. PS63B

Example 2 shows the aminoterminal and internal polypeptide sequences of the PS63B toxin protein as determined by standard Edman protein sequencing. From these sequences, two oligonucleotide primers were designed using a codon frequency table assembled from B.t. genes encoding δ-endotoxins. The sequence of the forward primer (63B-A) was complementary to the predicted DNA sequence at the 5' end of the gene: 63B-A - 5′ CAA T/CTA CAA GCA/T CAA CC 3′ The sequence of the reverse primer (63B-INT) was complementary to the inverse of the internal predicted DNA sequence: 63B-INT - 5′ TTC ATC TAA AAT TCT TTG A/TAC 3′ These primers were used in standard polymerase chain reactions (Cetus Corporation) to amplify an approximately 460 bp fragment of the 63B toxin gene for use as a DNA cloning probe. Standard Southern blots of total cellular DNA from PS63B were hybridized with the radiolabeled PCR probe. Hybridizing bands included an approximately 4.4 kbp XbaI fragment, an approximately 2.0 kbp HindIII fragment, and an approximately 6.4 kbp SpeI fragment.

### Example 4 ― Activity of the B.t. Toxin Protein and Gene Product Against Caenorhabditis elegans

Caenorhabditis elegans (CE) was cultured as described by Simpkin and Coles (J. Chem. Tech. Biotechnol. 31:66-69, 1981) in corning (Corning Glass Works, Corning, NY) 24-well tissue culture plates containing 1ml S-basal media, 0.5mg ampicillin and 0.01mg cholesterol. Each well also contained ca. 10⁸ cells of Escherichia coli strain OP-50, a uracil auxotroph. The wells were seeded with ca. 100-200 CE per well and incubated at 20°C. Samples of protein (obtained from the wild type B.t. or the recombinant B.t.) were added to the wells by serial dilution. Water served as the control as well as the vehicle to introduce the proteins to the wells.

Each of the wells were examined daily and representative results are as follows:

| | % KILL | | |
|---|---|---|---|
| µg toxin | pMYC2309 | pMYC2311 | PS17 |
| 100 | 25 | 50 | 75 |
| 32 | 25 | 50 | 75 |
| 10 | 50 | 25 | 50 |
| 1 | 0 | 0 | 0 |

### Example 5 ― Activity Against the Plant Nematodes Pratylenchus spp.

The toxin encoded by B.t. PS17a has been found to be active against Pratylenchus spp. The activity is approximately at the same level as is disclosed in Example 4 against C. elegans.

Species of Pratylenchus, for example, P. scribneri and P. redivivous, are known pathogens of many economically important crops including corn, peanuts, soybean, alfalfa, beans, tomato, and citrus. These "root lesion" nematodes are the second most economically damagging genus of plant parasitic nematodes (after Meliodogyne―the "root knot" nematode), and typify the migratory endoparasites.

Pratylenchus spp. was reared aseptically on excised corn roots in Gamburg's B5 medium (GIBCO® Laboratories, Grand Island, NY) Bioassays were done in 24 well assay plates (Corning #25820) using L 3-4 larvae as described by Tsai and van Gundy (J. Nematol. 22(3):327-332). Approximately 20 nematodes were placed in each well. A total of 80-160 nematodes were used in each treatment. Samples of protein were suspended in an aqueous solution using a hand-held Dounce homogenizer.

Mortality was assessed visually 3 or 7 days after treatment. Larvae that were nearly straight and not moving or not responding to prodding with a dull probe were considered moribund. Representative results are as follows:

| Toxin | Rate (ppm) | Days After Treatment | % Moribund |
|---|---|---|---|
| PS33F2 | 75 | 3 | 78 |
| | 0 3 | | 12 |
| | | | |
| PS52A1 | 200 | 7 | 75 |
| | 0 | 7 | 5 |

### Example 6 ― Activity of Bacillus thuringiensis Isolates Against Panagrellus redivivus

Worms are collected in a tube and allowed to settle for about 15 minutes, and the water is decanted and replaced with fresh water three or four times until the water remains clear. 250 *µ*l rinsed nematodes (20-30 worms), and 100 *µ*l of a spore/crystal suspension are added to 650 *µ*l water in each well of tray. Nematodes are counted and the numbers recorded. After four days, the live worms are counted and percent mortality is calculated.

| Bioassay Results: | |
|---|---|
| Prior art (U.S.S.N. 084,653, filed August 12, 1987) | |

| B.t. strain No. | Mortality |
|---|---|
| PS17 | 90% |
| PS33F2 | 30% |
| PS52A1 | 100% |
| PS63B | 92% |
| PS69D1 | 100% |

| Novel B.t. strain No. | |
|---|---|
| PS80JJ1 | 99% |
| PS158D5 | 99% |
| PS167P | 96% |
| PS169E | 100% |
| PS177F1 | 96% |
| PS177G | 100% |
| PS204G4 | 100% |
| PS204G6 | 100% |
| Control | 0% |

The following table shows the molecular mass of the alkali-soluble proteins in each novel nematode-active strain, as compared to prior art B.t. strains.

| Prior Art Nematode-Active Strains | |
|---|---|
| *B*.*t*. Strain | Approximate Molecular Mass of Proteins (kDa) |
| PS17 | 155, 145, 135 |
| PS33F2 | 140, 94, 86, 68, 65, 62 |
| PS52A1 | 57, 45 |
| PS63B | 84, 82, 78 |
| PS69D1 | 135, 46, 32 |

| New Nematode-Active Strains | |
|---|---|
| Novel *B.t.* Strain | Approximate Molecular Mass of Proteins (kDa) |
| PS80JJ1 | 130, 90, 47, 37 |
| PS158D5 | 80 |
| PS167P | 120 |
| PS169E | 150, 128, 33 |
| PS177F1 | 140, 116, 103, 62 |
| PS177G | 135, 125, 107, 98, 62 |
| PS204G4 | 105, 98, 90, 60, 44, 37 |
| PS204G6 | 23, 21 |

### Example 7 ― Activity Against Fasciola hepatica

Toxins produced by wild type B.t. isolates and recombinant microbes expressing B.t. proteins were tested for their flukicidal activity.

In vitro culture (37°C, 5% CO₂) was according to a modification of the method of Ibarra and Jenkins (Z. Parasitenkd. 70:655-661, 1984). Culture media consisted of RPMI (7.5 pH) with 50% v/v rabbit serum and 2% v/v rabbit RBC.

Test compounds. In the experiments described below, the effects of various substances on liver flukes were tested and compared. As used herein, Compound PS17 refers to toxin recovered and purified from cultures of B.t. PS17 as described above. Compound PS17a refers to toxin recovered and purified from cultures of the recombinant microbe which has been transformed with the B.t. gene designated B.t. PS17a. Compound C, is a formulation blank used as a negative control. ABZ refers to the drug Albendazole, which can be obtained from SmithKline Beecham, Lincoln, NE. Compounds PS17, PS17a, and C were added directly to media at 100 ppm; ABZ was dissolved in 100 *µ*l absolute ethanol prior to addition to media.

Criteria for efficacy. Flukes were examined hourly for 3-8 hours and then once or twice daily for effects of drug treatment as evidenced by death, motility disturbances, or morphologic changes as compared to untreated control flukes, using an inverted microscope at 40X.

Three-week-old Fasciola hepatica removed from the livers of experimentally infected rabbits were cultured in vitro for 5 days in 1.6 cm Linbro culture plate wells (2 ml media; 4-6 flukes/well). After 5 days incubation, flukes were transferred to media containing Compound PS17a (100 ppm; 5 flukes), Compound PS17 (100 ppm; 6 flukes), or to untreated control media (4 flukes). All flukes were dead in Compound PS17a by 18 hours. In Compound PS17 only one fluke was alive (sluggish) at 24 hours compared to three live flukes in the media control (Table 1). All flukes were fixed and stained for morphologic study; no apparent microscopic changes were observed except those attributable to analysis of dead flukes. During the 5-day pre-culture period, flukes remained active and were observed ingesting RBC's from the media, with an apparently active digestive function.

**Table 1.**

| Number of living immature F. hepatica (3 weeks old), rabbit origin | | | |
|---|---|---|---|
| Observation time | Compound PS17a | Compound PS17 | Media control |
| | 100 ppm | 100 ppm | |
| 0 | 5 | 6 | 4 |
| 5 minutes | 5 | 6 | 4 |
| 1 hour | 5 | 6 | 4 |
| 2 hours | 5 | 6 | 4 |
| 3 hours | 5 | 6 | 4 |
| 6 hours | 5 | 6 | 4 |
| 8 hours | 5 | 6 | 4 |
| 18 hours | 0 | 4 (sluggish) | 3 |
| 24 hours | | 1 (sluggish) | 3 |

Four mature flukes recovered from a naturally infected calf were separately cultured in vitro in 25 cm² tissue culture flasks in 5 ml media for 24 hours and then transferred to similar flasks containing Compound PS17a (100 ppm), Compound PS17 (100 ppm), ABZ (10 ppm) or untreated media (Table 2). Flukes in Compound PS17a died between 10 and 18 hours, in Compound PS17 between 18 and 20 hours, and in ABZ by 64 hours. The media control fluke died in 72 hours in association with contamination of media as evidenced by turbidity and yellow media coloration.

These flukes were cultured in RPMI 49% + Rabbit serum 49% + Rabbit RBC 2% for 5 days prior to trial initiation. The experiment terminated after 24 hours, and all flukes were fixed for morphological study. Behavior of flukes prior to mortality included less activity and oral suckers directed to the surface of the media. At death, the body is contracted, then relaxed prior to decomposition.

**Table 2.**

| Number of living mature flukes | | | | |
|---|---|---|---|---|
| Observation time | Compound PS17a | Compound PS17 | Albendazole | Media Control |
| | 100 ppm | 100 ppm | 10 ppm | |
| 0 | 1 | 1 | 1 | 1 |
| 5 min. | 1 | 1 | 1 | 1 |
| 1 hour | 1 | 1 | 1 | 1 |
| 2 hours | 1 | 1 | 1 | 1 |
| 3 hours | 1 | 1 | 1 | 1 |
| 6 hours | 1 | 1 | 1 | 1 |
| 8 hours | 1 | 1 | 1 | 1 |
| 10 hours | 1 | 1 | 1 | 1 |
| 18 hours | 0 | 1* | 1 | 1 |
| 20 hours | | 0 | 1 | 1 |
| 31 hours | | | 1 | 1 |
| 60 hours | | | 1 | 1 |
| 64 hours | | | 0 | 1 |
| 72 hours | | | | 0** |

| | | | | |
|---|---|---|---|---|
| * Sluggish movement. | | | | |
| ** Contracted folded body without movement, with turbidity of the media. | | | | |

### Example 8

An additional experiment was done to test the efficacy of Compounds PS17 and PS17a against F. hepatica as compared to albendazole (ABZ), a drug with known efficacy (positive control) and an untreated media control. Tests were done against 1) mature flukes recovered from naturally infected calves and 2) immature 5-week-old rabbit origin flukes.

Immature flukes. Five-week-old immature F. hepatica were recovered from experimentally infected rabbit livers and placed in culture media overnight (1.6 cm Linbro culture plates, 2-3 flukes per well) in triplicate. Flukes were then transferred to similar Linbro plates containing 2 ml of Compound PS17a (100 ppm), Compound PS17 (100 ppm), albendazole (ABZ, 10 ppm), or untreated media (Table 3). In Compound PS17a, 7 of 7 flukes had died by 20 hours; sluggish movement was noted at 8-hours-post-exposure. In Compound PS17, mortality (2 of 7 flukes) and sluggish movement of remaining flukes was noted at 20 hours; at 31 hours 5 of 7 flukes were dead; at 48 hours all flukes were dead. In the ABZ positive control, 1 of 7 flukes was dead at 20 hours, 4 had died by 31 hours, and remaining flukes died by 60 hours. In untreated control media, 2 of 7 flukes had died at 31 hours, 3 at 48 hours, 4 at 55 hours, 5 at 60 hours, and 7 of 7 were dead by 72 hours. (NOTE: Superficial layers of some wells became turbid at 35 hours post-exposure; all flukes were washed in RPMI and transferred to wells containing fresh media at original drug concentrations).

Mature flukes. Mature flukes recovered from 3 calf livers that had been condemned due to fascioliasis (Roucher's Meat Packing, Plaquemine, LA) were washed and held in sterile saline (for 2-3 hours) and four flukes were placed in each of two 25 ml tissue culture flasks containing Compound PS17a (100 ppm); Compound PS17 (100 ppm); ABZ (10 ppm) or untreated control media (Table 4). All flukes were dead in Compound PS17a by 10-11 hours and in Compound PS17 by 20 hours. In ABZ, flukes died by 48-54 hours. In control media, 2 flukes died by 48 hours; all flukes died by 66 hours. (NOTE: Contamination in flasks was noted at 31-48 hours).

**Table 3.**

| Number of living immature flukes, rabbit origin (5 weeks old) | | | | | | | | | | | | |
|---|---|---|---|---|---|---|---|---|---|---|---|---|
| Obser. time | Compound PS17a 100 ppm | | | Compound PS17 100 ppm | | | Albendazole 10 ppm | | | Media control | | |
| | W1 | W2 | W3 | W1 | W2 | W3 | W1 | W2 | W3 | W1 | W2 | W3 |
| 0 | 2 | 2 | 3 | 2 | 2 | 3 | 2 | 2 | 2 | 2 | 2 | 3 |
| 5 min. | 2 | 2 | 3 | 2 | 2 | 3 | 2 | 2 | 2 | 2 | 2 3 | |
| 1 hr. | 2 | 2 | 3 | 2 | 2 | 3 | 2 | 2 | 2 | 2 | 2 | 3 |
| 2 hr. | 2 | 2 | 3 | 2 | 2 | 3 | 2 | 2 | 2 | 2 | 2 | 3 |
| 3 hr. | 2 | 2 | 3 | 2 | 2 | 3 | 2 | 2 | 2 | 2 | 2 | 3 |
| 6 hr. | 2 | 2 | 3 | 2 | 2 | 3 | 2 | 2 | 2 | 2 | 2 | 3 |
| 8 hr. | 2* | 2* | 3* | 2 | 2 | 3 | 2 | 2 | 2 | 2 | 2 | 3 |
| 20 hr. | 0 | 0 | 0 | 2* | 2* | 1* | 2 | 2 | 2 | 2 | 2 | 3 |
| 31 hr. | | | | 1* | 0 | 1* | 1 | 1 | 1 | 2 | 2 | 1 |
| 35 hr. Note: Superficial layer of some wells appeared turbid. Media was changed and the flukes washed in RPMI only. Washed living flukes were transferred to new media with drugs at original concentrations. | | | | | | | | | | | | |
| 48 hr. | | | | 0 | 0 | 0 | 1 | 1 | 1 | 1 | 2 | 1 |
| 55 hr. | | | | | | | 1 | 1 | 1 | 1 | 2 | |
| 60 hr. | | | | | | | 0 | 0 | 0 | 1* | 1* | 0 |
| 72 hr. | | | | | | | | | | 0 | 0 | 0 |

| | | | | | | | | | | | | |
|---|---|---|---|---|---|---|---|---|---|---|---|---|
| * Sluggish flukes. | | | | | | | | | | | | |

**Table 4.**

| Effect on mature flukes (cattle origin) | | | | | | | | |
|---|---|---|---|---|---|---|---|---|
| Obser. time | Compound PS17a 100 ppm | | Compound PS17 100 ppm | | Albendazole | | Media | control |
| | Flask 1 | Flask 2 | Flask 1 | Flask 2 | Flask 1 | Flask 2 | Flask 1 | Flask 2 |
| 0 | 4 | 4 | 4 | 4 | 4 | 4 | 4 | 4 |
| 5 min. | 4 | 4 | 4 | 4 | 4 | 4 | 4 | 4 |
| 1 hr. | 4 | 4 | 4 | 4 | 4 | 4 | 4 | 4 |
| 2 hr. | 4 | 4 | 4 | 4 | 4 | 4 | 4 | 4 |
| 3 hr. | 4 | 4 | 4 | 4 | 4 | 4 | 4 | 4 |
| 8 hr. | 4 | 4 | 4 | 4 | 4 | 4 | 4 | 4 |
| 10-11 hr. | 0 | 0 | 4 | 4 | 4 | 4 | 4 | 4 |
| 18 hr. | | | 4* | 4* | 4 | 4 | 4 | 4 |
| 20 hr. | | | 0 | 0 | 4 | 4 | 4 | 4 |
| 31 hr. | | | | | 4 | 4 | 4 | 4 |
| 48 hr. | | | | | 2 | 3 | 4 | 2 |
| Note: Apparent contamination in flasks between 31-48 hours | | | | | | | | |
| 54 hr. | | | | | 0 | 0 | 3 | 1 |
| 66 hr. | | | | | | | 0 | 0 |

| | | | | | | | | |
|---|---|---|---|---|---|---|---|---|
| * Sluggish movement. | | | | | | | | |

### Example 9

Flukes were recovered from bile ducts of condemned calf livers and washed in sterile RPMI for 2-3 hours in groups of 10-20 flukes. Only one fluke was cultured in each well of 6-well Linbro tissue culture plates (10 ml size), with each well containing 4 ml treated or untreated media. A formulation blank (Compound C) having no known flukicidal activity was also tested. Additional controls were added to identify sources of potential contamination, including a media control with 2% RBC and a media control without 2% RBC. The ABZ concentration was increased to 25 ppm. Control wells containing drug or media only were also included to control for contamination source.

This experiment included 12 replicates (Table 5); six of these replicates had corresponding wells with treated or untreated media only. For Compound PS17a, all 12 flukes died by 12 hours; weak movement was observed as early as 10 hours. All Compound PS17 flukes were dead by the 19 or 24 hour observation. Compound C fluke mortality was observed at 36-58 hours. For the media with 2% RBC and media without 2% RBC controls, one fluke was dead at 36 hours, two at 58 hours, and three at 72 hours; one fluke survived to 115 hours in media with 2% RBC.

For 8 of the 12 replicates and two of the 6 corresponding media controls, the media was electively changed in both fluke containing and non-fluke containing wells at 24 hours post-exposure. This extra handling procedure may have resulted in death of all flukes by 58 hours in 6 of the 8 replicates, apparently due to contamination as evidenced by turbidity and yellow color of media. Two fluke replicates in which media was changed in 24 hours did not become contaminated. None of the six media control (no fluke) replicates became contaminated during 115 hours of incubation.

**Table 5.**

| *In vitro* efficacy against adult *Fasciola hepatica* (cattle origin) | | | | | | |
|---|---|---|---|---|---|---|
| 'Time Hr. | Compound PS17a 100 ppm | Compound PS17 100 ppm | Compound C 100 ppm | ABZ 25 ppm | Media No RBC | Media RBC |
| 1 | 12 | 12 | 12 | 12 | 12 | 12 |
| 2 | 12 | 12 | 12 | 12 | 12 | 12 |
| 3 | 12 | 12 | 12 | 12 | 12 | 12 |
| 10 | 12* | 12 | 12 | 12 | 12 | 12 |
| 14 | 0 | 12 | 12 | 12 | 12 | 12 |
| 19 | | 10 | 12 | 12 | 12 | 12 |
| 24 | | 0 | 12 | 11 | 12 | 12 |
| 36 | | | 8 (5)† | 7 (4) | 11 (5) | 11 (5) |
| 44 | | | 4 (3) | 5 (2) | 11 (5) | 11 (5) |
| 58** | | | 0 (0) | 2 (0) | 5 (0) | 5 (0) |
| 74 | | | | 1 | 4 | 4 |
| 98 | | | | 0 | 1 | 2 |
| 115 | | | | | 0 | 1 |

| | | | | | | |
|---|---|---|---|---|---|---|
| * Weak movement only. | | | | | | |
| † (Number of live flukes that were in replicates receiving media change at 24 hr.). | | | | | | |
| ** All flukes in 6 of 8 replicates that received media change at 24 hr. were dead at 58 hours due to contamination, probably related to additional handling. Two media control replicates changed at 24 hours were not contaminated. | | | | | | |

### Example 10 ― Insertion of Toxin Gene Into Plants

The novel gene coding for the novel nematode-active toxin, as disclosed herein, can be inserted into plant cells using the Ti plasmid from Agrobacter tumefaciens. Plant cells can then be caused to regenerate into plants (Zambryski, P., Joos, H., Gentello, C., Leemans, J., Van Montague, M. and Schell, J [1983] Cell 32:1033-1043). A particularly useful vector in this regard is pEND4K (Klee, H.J., Yanofsky, M.F. and Nester, E.W. [1985] Bio/Technology 3:637-642). This plasmid can replicate both in plant cells and in bacteria and has multiple cloning sites for passenger genes. The toxin gene, for example, can be inserted into the BamHI site of pEND4K, propagated in E. coli, and transformed into appropriate plant cells.

### Example 11 ― Cloning of Novel Bacillus thuringiensis Gene Into Baculoviruses

The novel gene of the invention can be cloned into baculoviruses such as Autographa californica nuclear polyhedrosis virus (AcNPV). Plasmids can be constructed that contain the AcNPV genome cloned into a commercial cloning vector such as pUC8. The AcNPV genome is modified so that the coding region of the polyhedrin gene is removed and a unique cloning site for a passenger gene is placed directly behind the polyhedrin promoter. Examples of such vectors are pGP-B6874, described by Pennock et al. (Pennock, G.D., Shoemaker, C. and Miller, L.K. [1984] Mol. Cell. Biol. 4:399-406), and pAC380, described by Smith et al. (Smith, G.E., Summers, M.D. and Fraser, M.J. [1983] Mol Cell. Biol. 3:2156-2165). The gene coding for the novel protein toxin of the invention can be modified with BamHI linkers at appropriate regions both upstream and downstream from the coding region and inserted into the passenger site of one of the AcNPV vectors.

It should be understood that the examples and embodiments described herein are for illustrative purposes only and that various modifications or changes in light thereof will be suggested to persons skilled in the art and are to be included within the spirit and purview of this application and the scope of the appended claims.

SEQUENCE LISTING

<110> Mycogen Corporation

<120> Novel Bacillus thuringiensis Microbes Active Against Nematodes, and
Genes Encoding Novel Nematode-Active Toxins Cloned from Bacillus thuringiensis
Isolates

<130> MA20C-48DIV1 EPO

<140>
<141>

<150> 91305047.2
<151> 1991-06-04

<160> 10

<170> PatentIn Ver. 2.1

<210> 1
<211> 4155
<212> DNA
<213> Bacillus thuringiensis

<400> 1
atggcaattt taaatgaatt atatccatct gtaccttata atgtattggc gtatacgcca 60
ccctcttttt tacctgatgc gggtacacaa gctacacctg ctgacttaac agcttatgaa 120
caattgttga aaaatttaga aaaagggata aatgctggaa cttattcgaa agcaatagct 180
gatgtactta aaggtatttt tatagatgat acaataaatt atcaaacata tgtaaatatt 240
ggtttaagtt taattacatt agctgtaccg gaaattggta tttttacacc tttcatcggt 300
ttgttttttg ctgcattgaa taaacatgat gctccacctc ctcctaatgc aaaagatata 360
tttgaggcta tgaaaccagc gattcaagag atgattgata gaactttaac tgcggatgag 420
caaacatttt taaatgggga aataagtggt ttacaaaatt tagcagcaag ataccagtct 480
acaatggatg atattcaaag ccatggagga tttaataagg tagattctgg attaattaaa 540
aagtttacag atgaggtact atctttaaat agtttttata cagatcgttt acctgtattt 600
attacagata atacagcgga tcgaactttg ttaggtcttc cttattatgc tatacttgcg 660
agcatgcatc ttatgttatt aagagatatc attactaagg gtccgacatg ggattctaaa 720
attaatttca caccagatgc aattgattcc tttaaaaccg atattaaaaa taatataaag 780
ctttactcta aaactattta tgacgtattt cagaagggac ttgcttcata cggaacgcct 840
tctgatttag agtcctttgc aaaaaaacaa aaatatattg aaattatgac aacacattgt 900
ttagattttg caagattgtt tcctactttt gatccagatc tttatccaac aggatcaggt 960
gatataagtt tacaaaaaac acgtagaatt ctttctcctt ttatccctat acgtactgca 1020
gatgggttaa cattaaataa tacttcaatt gatacttcaa attggcctaa ttatgaaaat 1080
gggaatggcg cgtttccaaa cccaaaagaa agaatattaa aacaattcaa actgtatcct 1140
agttggagag cgggacagta cggtgggctt ttacaacctt atttatgggc aatagaagtc 1200
caagattctg tagagactcg tttgtatggg cagcttccag ctgtagatcc acaggcaggg 1260

cctaattatg tttccataga ttcttctaat ccaatcatac aaataaatat ggatacttgg 1320
aaaacaccac cacaaggtgc gagtgggtgg aatacaaatt taatgagagg aagtgtaagc 1380
gggttaagtt ttttacaacg agatggtacg agacttagtg ctggtatggg tggtggtttt 1440
gctgatacaa tatatagtct ccctgcaact cattatcttt cttatctcta tggaactcct 1500
tatcaaactt ctgataacta ttctggtcac gttggtgcat tggtaggtgt gagtacgcct 1560
caagaggcta ctcttcctaa tattataggt caaccagatg aacagggaaa tgtatctaca 1620
atgggatttc cgtttgaaaa agcttcttat ggaggtacag ttgttaaaga atggttaaat 1680
ggtgcgaatg cgatgaagct ttctcctggg caatctatag gtattcctat tacaaatgta 1740
acaagtggag aatatcaaat tcgttgtcgt tatgcaagta atgataatac taacgttttc 1800
tttaatgtag atactggtgg agcaaatcca attttccaac agataaactt tgcatctact 1860
gtagataata atacgggagt acaaggagca aatggtgtct atgtagtcaa atctattgct 1920
acaactgata attcttttac agaaattcct gcgaagacga ttaatgttca tttaaccaac 1980
caaggttctt ctgatgtctt tttagaccgt attgaattta tacctttttc tctacctctt 2040
atatatcatg gaagttataa tacttcatca ggtgcagatg atgttttatg gtcttcttca 2100
aatatgaatt actacgatat aatagtaaat ggtcaggcca atagtagtag tatcgctagt 2160
tctatgcatt tgcttaataa aggaaaagtg ataaaaacaa ttgatattcc agggcattcg 2220
gaaaccttct ttgctacgtt cccagttcca gaaggattta atgaagttag aattcttgct 2280
ggccttccag aagttagtgg aaatattacc gtacaatcta ataatccgcc tcaacctagt 2340
aataatggtg gtggtgatgg tggtggtaat ggtggtggtg atggtggtca atacaatttt 2400
tctttaagcg gatctgatca tacgactatt tatcatggaa aacttgaaac tgggattcat 2460
gtacaaggta attataccta tacaggtact cccgtattaa tactgaatgc ttacagaaat 2520
aatactgtag tatcaagcat tccagtatat tctccttttg atataactat acagacagaa 2580
gctgatagcc ttgagcttga actacaacct agatatggtt ttgccacagt gaatggtact 2640
gcaacagtaa aaagtcctaa tgtaaattac gatagatcat ttaaactccc aatagactta 2700
caaaatatca caacacaagt aaatgcatta ttcgcatctg gaacacaaaa tatgcttgct 2760
cataatgtaa gtgatcatga tattgaagaa gttgtattaa aagtggatgc cttatcagat 2820
gaagtatttg gagatgagaa gaaggcttta cgtaaattgg tgaatcaagc aaaacgtttg 2880
agtagagcaa gaaatcttct gataggtggg agttttgaaa attgggatgc atggtataaa 2940
ggaagaaatg tagtaactgt atctgatcat gaactattta agagtgatca tgtattatta 3000
ccaccaccag gattgtctcc atcttatatt ttccaaaaag tggaggaatc taaattaaaa 3060
ccaaatacac gttatattgt ttctggattc atcgcacatg gaaaagacct agaaattgtt 3120
gtttcacgtt atgggcaaga agtgcaaaag gtcgtgcaag ttccttatgg agaagcattc 3180
ccgttaacat caaatggacc agtttgttgt cccccacgtt ctacaagtaa tggaacctta 3240
ggagatccac atttctttag ttacagtatc gatgtaggtg cactagattt acaagcaaac 3300
cctggtattg aatttggtct tcgtattgta aatccaactg gaatggcacg cgtaagcaat 3360
ttggaaattc gtgaagatcg tccattagca gcaaatgaaa tacgacaagt acaacgtgtc 3420
gcaagaaatt ggagaaccga gtatgagaaa gaacgtgcgg aagtaacaag tttaattcaa 3480
cctgttatca atcgaatcaa cggattgtat gaaaatggaa attggaacgg ttctattcgt 3540
tcagatattt cgtatcagaa tatagacgcg attgtattac caacgttacc aaagttacgc 3600
cattggttta tgtcagatag attcagtgaa caaggagata taatggctaa attccaaggt 3660
gcattaaatc gtgcgtatgc acaactggaa caaagtacgc ttctgcataa tggtcatttt 3720
acaaaagatg cagctaattg gacaatagaa ggcgatgcac atcagataac actagaagat 3780
ggtagacgtg tattgcgact tccagattgg tcttcgagtg tatctcaaat gattgaaatc 3840
gagaatttta atccagataa agaatacaac ttagtattcc atgggcaagg agaaggaacg 3900
gttacgttgg agcatggaga agaaacaaaa tatatagaaa cgcatacaca tcattttgcg 3960
aattttacaa cttctcaacg tcaaggactc acgtttgaat caaataaagt gacagtgacc 4020
atttcttcag aagatggaga attcttagtg gataatattg cgcttgtgga agctcctctt 4080
cctacagatg accaaaattc tgagggaaat acggcttcca gtacgaatag cgatacaagt 4140

atgaacaaca atcaa 4155

<210> 2
<211> 1385
<212> PRT
<213> Bacillus thuringiensis

<400> 2
Met Ala Ile Leu Asn Glu Leu Tyr Pro Ser Val Pro Tyr Asn Val Leu
1               5                   10                  15

Ala Tyr Thr Pro Pro Ser Phe Leu Pro Asp Ala Gly Thr Gln Ala Thr
            20                  25                  30

Pro Ala Asp Leu Thr Ala Tyr Glu Gln Leu Leu Lys Asn Leu Glu Lys
        35                  40                  45

Gly Ile Asn Ala Gly Thr Tyr Ser Lys Ala Ile Ala Asp Val Leu Lys
    50                  55                  60

Gly Ile Phe Ile Asp Asp Thr Ile Asn Tyr Gln Thr Tyr Val Asn Ile
65                  70                  75                  80

Gly Leu Ser Leu Ile Thr Leu Ala Val Pro Glu Ile Gly Ile Phe Thr
                85                  90                  95

Pro Phe Ile Gly Leu Phe Phe Ala Ala Leu Asn Lys His Asp Ala Pro
            100                 105                 110

Pro Pro Pro Asn Ala Lys Asp Ile Phe Glu Ala Met Lys Pro Ala Ile
        115                 120                 125

Gln Glu Met Ile Asp Arg Thr Leu Thr Ala Asp Glu Gln Thr Phe Leu
    130                 135                 140

Asn Gly Glu Ile Ser Gly Leu Gln Asn Leu Ala Ala Arg Tyr Gln Ser
145                 150                 155                 160

Thr Met Asp Asp Ile Gln Ser His Gly Gly Phe Asn Lys Val Asp Ser
                165                 170                 175

Gly Leu Ile Lys Lys Phe Thr Asp Glu Val Leu Ser Leu Asn Ser Phe
            180                 185                 190

Tyr Thr Asp Arg Leu Pro Val Phe Ile Thr Asp Asn Thr Ala Asp Arg
        195                 200                 205

Thr Leu Leu Gly Leu Pro Tyr Tyr Ala Ile Leu Ala Ser Met His Leu
    210                 215                 220

Met Leu Leu Arg Asp Ile Ile Thr Lys Gly Pro Thr Trp Asp Ser Lys
225                 230                 235                 240

Ile Asn Phe Thr Pro Asp Ala Ile Asp Ser Phe Lys Thr Asp Ile Lys
                245                 250                 255

Asn Asn Ile Lys Leu Tyr Ser Lys Thr Ile Tyr Asp Val Phe Gln Lys
            260                 265                 270

Gly Leu Ala Ser Tyr Gly Thr Pro Ser Asp Leu Glu Ser Phe Ala Lys
        275                 280                 285

Lys Gln Lys Tyr Ile Glu Ile Met Thr Thr His Cys Leu Asp Phe Ala
    290                 295                 300

Arg Leu Phe Pro Thr Phe Asp Pro Asp Leu Tyr Pro Thr Gly Ser Gly
305                 310                 315                 320

Asp Ile Ser Leu Gln Lys Thr Arg Arg Ile Leu Ser Pro Phe Ile Pro
                325                 330                 335

Ile Arg Thr Ala Asp Gly Leu Thr Leu Asn Asn Thr Ser Ile Asp Thr
            340                 345                 350

Ser Asn Trp Pro Asn Tyr Glu Asn Gly Asn Gly Ala Phe Pro Asn Pro
        355                 360                 365

Lys Glu Arg Ile Leu Lys Gln Phe Lys Leu Tyr Pro Ser Trp Arg Ala
    370                 375                 380

Gly Gln Tyr Gly Gly Leu Leu Gln Pro Tyr Leu Trp Ala Ile Glu Val
385                 390                 395                 400

Gln Asp Ser Val Glu Thr Arg Leu Tyr Gly Gln Leu Pro Ala Val Asp
                405                 410                 415

Pro Gln Ala Gly Pro Asn Tyr Val Ser Ile Asp Ser Ser Asn Pro Ile
            420                 425                 430

Ile Gln Ile Asn Met Asp Thr Trp Lys Thr Pro Pro Gln Gly Ala Ser
        435                 440                 445

Gly Trp Asn Thr Asn Leu Met Arg Gly Ser Val Ser Gly Leu Ser Phe
    450                 455                 460

Leu Gln Arg Asp Gly Thr Arg Leu Ser Ala Gly Met Gly Gly Gly Phe
465                 470                 475                 480

Ala Asp Thr Ile Tyr Ser Leu Pro Ala Thr His Tyr Leu Ser Tyr Leu
                485                 490                 495

Tyr Gly Thr Pro Tyr Gln Thr Ser Asp Asn Tyr Ser Gly His Val Gly
            500                 505                 510

Ala Leu Val Gly Val Ser Thr Pro Gln Glu Ala Thr Leu Pro Asn Ile
        515                 520                 525

Ile Gly Gln Pro Asp Glu Gln Gly Asn Val Ser Thr Met Gly Phe Pro
    530                 535                 540

Phe Glu Lys Ala Ser Tyr Gly Gly Thr Val Val Lys Glu Trp Leu Asn
545                 550                 555                 560

Gly Ala Asn Ala Met Lys Leu Ser Pro Gly Gln Ser Ile Gly Ile Pro
                565                 570                 575

Ile Thr Asn Val Thr Ser Gly Glu Tyr Gln Ile Arg Cys Arg Tyr Ala
            580                 585                 590

Ser Asn Asp Asn Thr Asn Val Phe Phe Asn Val Asp Thr Gly Gly Ala
        595                 600                 605

Asn Pro Ile Phe Gln Gln Ile Asn Phe Ala Ser Thr Val Asp Asn Asn
    610                 615                 620

Thr Gly Val Gln Gly Ala Asn Gly Val Tyr Val Val Lys Ser Ile Ala
625                 630                 635                 640

Thr Thr Asp Asn Ser Phe Thr Glu Ile Pro Ala Lys Thr Ile Asn Val
                645                 650                 655

His Leu Thr Asn Gln Gly Ser Ser Asp Val Phe Leu Asp Arg Ile Glu
            660                 665                 670

Phe Ile Pro Phe Ser Leu Pro Leu Ile Tyr His Gly Ser Tyr Asn Thr
        675                 680                 685

Ser Ser Gly Ala Asp Asp Val Leu Trp Ser Ser Ser Asn Met Asn Tyr
    690                 695                 700

Tyr Asp Ile Ile Val Asn Gly Gln Ala Asn Ser Ser Ser Ile Ala Ser
705                 710                 715                 720

Ser Met His Leu Leu Asn Lys Gly Lys Val Ile Lys Thr Ile Asp Ile
                725                 730                 735

Pro Gly His Ser Glu Thr Phe Phe Ala Thr Phe Pro Val Pro Glu Gly
            740                 745                 750

Phe Asn Glu Val Arg Ile Leu Ala Gly Leu Pro Glu Val Ser Gly Asn
        755                 760                 765

Ile Thr Val Gln Ser Asn Asn Pro Pro Gln Pro Ser Asn Asn Gly Gly
    770                 775                 780

Gly Asp Gly Gly Gly Asn Gly Gly Gly Asp Gly Gly Gln Tyr Asn Phe
785                 790                 795                 800

Ser Leu Ser Gly Ser Asp His Thr Thr Ile Tyr His Gly Lys Leu Glu
                805                 810                 815

Thr Gly Ile His Val Gln Gly Asn Tyr Thr Tyr Thr Gly Thr Pro Val
            820                 825                 830

Leu Ile Leu Asn Ala Tyr Arg Asn Asn Thr Val Val Ser Ser Ile Pro
        835                 840                 845

Val Tyr Ser Pro Phe Asp Ile Thr Ile Gln Thr Glu Ala Asp Ser Leu
    850                 855                 860

Glu Leu Glu Leu Gln Pro Arg Tyr Gly Phe Ala Thr Val Asn Gly Thr
865                 870                 875                 880

Ala Thr Val Lys Ser Pro Asn Val Asn Tyr Asp Arg Ser Phe Lys Leu
                885                 890                 895

Pro Ile Asp Leu Gln Asn Ile Thr Thr Gln Val Asn Ala Leu Phe Ala
            900                 905                 910

Ser Gly Thr Gln Asn Met Leu Ala His Asn Val Ser Asp His Asp Ile
        915                 920                 925

Glu Glu Val Val Leu Lys Val Asp Ala Leu Ser Asp Glu Val Phe Gly
    930                 935                 940

Asp Glu Lys Lys Ala Leu Arg Lys Leu Val Asn Gln Ala Lys Arg Leu
945                 950                 955                 960

Ser Arg Ala Arg Asn Leu Leu Ile Gly Gly Ser Phe Glu Asn Trp Asp
                965                 970                 975

Ala Trp Tyr Lys Gly Arg Asn Val Val Thr Val Ser Asp His Glu Leu
            980                 985                 990

Phe Lys Ser Asp His Val Leu Leu Pro Pro Pro Gly Leu Ser Pro Ser
        995                 1000                1005

Tyr Ile Phe Gln Lys Val Glu Glu Ser Lys Leu Lys Pro Asn Thr Arg
    1010                1015                1020

Tyr Ile Val Ser Gly Phe Ile Ala His Gly Lys Asp Leu Glu Ile Val
1025                1030                1035                1040

Val Ser Arg Tyr Gly Gln Glu Val Gln Lys Val Val Gln Val Pro Tyr
                1045                1050                1055

Gly Glu Ala Phe Pro Leu Thr Ser Asn Gly Pro Val Cys Cys Pro Pro
            1060                1065                1070

Arg Ser Thr Ser Asn Gly Thr Leu Gly Asp Pro His Phe Phe Ser Tyr
        1075                1080                1085

Ser Ile Asp Val Gly Ala Leu Asp Leu Gln Ala Asn Pro Gly Ile Glu
    1090                1095                1100

Phe Gly Leu Arg Ile Val Asn Pro Thr Gly Met Ala Arg Val Ser Asn
1105                1110                1115                1120

Leu Glu Ile Arg Glu Asp Arg Pro Leu Ala Ala Asn Glu Ile Arg Gln
                1125                1130                1135

Val Gln Arg Val Ala Arg Asn Trp Arg Thr Glu Tyr Glu Lys Glu Arg
            1140                1145                1150

Ala Glu Val Thr Ser Leu Ile Gln Pro Val Ile Asn Arg Ile Asn Gly
        1155                1160                1165

Leu Tyr Glu Asn Gly Asn Trp Asn Gly Ser Ile Arg Ser Asp Ile Ser
    1170                1175                1180

Tyr Gln Asn Ile Asp Ala Ile Val Leu Pro Thr Leu Pro Lys Leu Arg
1185                1190                1195                1200

His Trp Phe Met Ser Asp Arg Phe Ser Glu Gln Gly Asp Ile Met Ala
                1205                1210                1215

Lys Phe Gln Gly Ala Leu Asn Arg Ala Tyr Ala Gln Leu Glu Gln Ser
            1220                1225                1230

Thr Leu Leu His Asn Gly His Phe Thr Lys Asp Ala Ala Asn Trp Thr
        1235                1240                1245

Ile Glu Gly Asp Ala His Gln Ile Thr Leu Glu Asp Gly Arg Arg Val
    1250                1255                1260

Leu Arg Leu Pro Asp Trp Ser Ser Ser Val Ser Gln Met Ile Glu Ile
1265                1270                1275                1280

Glu Asn Phe Asn Pro Asp Lys Glu Tyr Asn Leu Val Phe His Gly Gln
                1285                1290                1295

Gly Glu Gly Thr Val Thr Leu Glu His Gly Glu Glu Thr Lys Tyr Ile
            1300                1305                1310

Glu Thr His Thr His His Phe Ala Asn Phe Thr Thr Ser Gln Arg Gln
        1315                1320                1325

Gly Leu Thr Phe Glu Ser Asn Lys Val Thr Val Thr Ile Ser Ser Glu
    1330                1335                1340

Asp Gly Glu Phe Leu Val Asp Asn Ile Ala Leu Val Glu Ala Pro Leu
1345                1350                1355                1360

Pro Thr Asp Asp Gln Asn Ser Glu Gly Asn Thr Ala Ser Ser Thr Asn
                1365                1370                1375

Ser Asp Thr Ser Met Asn Asn Asn Gln
            1380                1385

<210> 3
<211> 3867
<212> DNA
<213> Bacillus thuringiensis

<400> 3
atggcaattt taaatgaatt atatccatct gtaccttata atgtattggc gtatacgcca 60
ccctcttttt tacctgatgc gggtacacaa gctacacctg ctgacttaac agcttatgaa 120
caattgttga aaaatttaga aaaagggata aatgctggaa cttattcgaa agcaatagct 180
gatgtactta aaggtatttt tatagatgat acaataaatt atcaaacata tgtaaatatt 240
ggtttaagtt taattacatt agctgtaccg gaaattggta tttttacacc tttcatcggt 300
ttgttttttg ctgcattgaa taaacatgat gctccacctc ctcctaatgc aaaagatata 360
tttgaggcta tgaaaccagc gattcaagag atgattgata gaactttaac tgcggatgag 420
caaacatttt taaatgggga aataagtggt ttacaaaatt tagcagcaag ataccagtct 480
acaatggatg atattcaaag ccatggagga tttaataagg tagattctgg attaattaaa 540
aagtttacag atgaggtact atctttaaat agtttttata cagatcgttt acctgtattt 600

attacagata atacagcgga tcgaactttg ttaggtcttc cttattatgc tatacttgcg 660
agcatgcatc ttatgttatt aagagatatc attactaagg gtccgacatg ggattctaaa 720
attaatttca caccagatgc aattgattcc tttaaaaccg atattaaaaa taatataaag 780
ctttactcta aaactattta tgacgtattt cagaagggac ttgcttcata cggaacgcct 840
tctgatttag agtcctttgc aaaaaaacaa aaatatattg aaattatgac aacacattgt 900
ttagattttg caagattgtt tcctactttt gatccagatc tttatccaac aggatcaggt 960
gatataagtt tacaaaaaac acgtagaatt ctttctcctt ttatccctat acgtactgca 1020
gatgggttaa cattaaataa tacttcaatt gatacttcaa attggcctaa ttatgaaaat 1080
gggaatggcg cgtttccaaa cccaaaagaa agaatattaa aacaattcaa actgtatcct 1140
agttggagag cggcacagta cggtgggctt ttacaacctt atttatgggc aatagaagtc 1200
caagattctg tagagactcg tttgtatggg cagcttccag ctgtagatcc acaggcaggg 1260
cctaattatg tttccataga ttcttctaat ccaatcatac aaataaatat ggatacttgg 1320
aaaacaccac cacaaggtgc gagtgggtgg aatacaaatt taatgagagg aagtgtaagc 1380
gggttaagtt ttttacaacg agatggtacg agacttagtg ctggtatggg tggtggtttt 1440
gctgatacaa tatatagtct ccctgcaact cattatcttt cttatctcta tggaactcct 1500
tatcaaactt ctgataacta ttctggtcac gttggtgcat tggtaggtgt gagtacgcct 1560
caagaggcta ctcttcctaa tattataggt caaccagatg aacagggaaa tgtatctaca 1620
atgggatttc cgtttgaaaa agcttcttat ggaggtacag ttgttaaaga atggttaaat 1680
ggtgcgaatg cgatgaagct ttctcctggg caatctatag gtattcctat tacaaatgta 1740
acaagtggag aatatcaaat tcgttgtcgt tatgcaagta atgataatac taacgttttc 1800
tttaatgtag atactggtgg agcaaatcca attttccaac agataaactt tgcatctact 1860
gtagataata atacgggagt acaaggagca aatggtgtct atgtagtcaa atctattgct 1920
acaactgata attcttttac agtaaaaatt cctgcgaaga cgattaatgt tcatttaacc 1980
aaccaaggtt cttctgatgt ctttttagat cgtattgagt ttgttccaat tctagaatca 2040
aatactgtaa ctatattcaa caattcatat actacaggtt cagcaaatct tataccagca 2100
atagctcctc tttggagtac tagttcagat aaagccctta caggttctat gtcaataaca 2160
ggtcgaacta cccctaacag tgatgatgct ttgcttcgat tttttaaaac taattatgat 2220
acacaaacca ttcctattcc gggttccgga aaagatttta caaatactct agaaatacaa 2280
gacatagttt ctattgatat ttttgtcgga tctggtctac atggatccga tggatctata 2340
aaattagatt ttaccaataa taatagtggt agtggtggct ctccaaagag tttcaccgag 2400
caaaatgatt tagagaatat cacaacacaa gtgaatgctc tattcacatc taatacacaa 2460
gatgcacttg caacagatgt gagtgatcat gatattgaag aagtggttct aaaagtagat 2520
gcattatctg atgaagtgtt tggaaaagag aaaaaaacat tgcgtaaatt tgtaaatcaa 2580
gcgaagcgct taagcaaggc gcgtaatctc ctggtaggag gcaattttga taacttggat 2640
gcttggtata gaggaagaaa tgtagtaaac gtatctaatc acgaactgtt gaagagtgat 2700
catgtattat taccaccacc aggattgtct ccatcttata ttttccaaaa agtggaggaa 2760
tctaaattaa aacgaaatac acgttatacg gtttctggat ttattgcgca tgcaacagat 2820
ttagaaattg tggtttctcg ttatgggcaa gaaataaaga aagtggtgca agttccttat 2880
ggagaagcat tcccattaac atcaagtgga ccagtttgtt gtatcccaca ttctacaagt 2940
aatggaactt taggcaatcc acatttcttt agttacagta ttgatgtagg tgcattagat 3000
gtagacacaa accctggtat tgaattcggt cttcgtattg taaatccaac tggaatggca 3060
cgcgtaagca atttggaaat tcgtgaagat cgtccattag cagcaaatga aatacgacaa 3120
gtacaacgtg tcgcaagaaa ttggagaacc gagtatgaga aagaacgtgc ggaagtaaca 3180
agtttaattc aacctgttat caatcgaatc aatggattgt atgacaatgg aaattggaac 3240
ggttctattc gttcagatat ttcgtatcag aatatagacg cgattgtatt accaacgtta 3300
ccaaagttac gccattggtt tatgtcagat agatttagtg aacaaggaga tatcatggct 3360
aaattccaag gtgcattaaa tcgtgcgtat gcacaactgg aacaaaatac gcttctgcat 3420
aatggtcatt ttacaaaaga tgcagccaat tggacggtag aaggcgatgc acatcaggta 3480

gtattagaag atggtaaacg tgtattacga ttgccagatt ggtcttcgag tgtgtctcaa 3540
acgattgaaa tcgagaattt tgatccagat aaagaatatc aattagtatt tcatgggcaa 3600
ggagaaggaa cggttacgtt ggagcatgga gaagaaacaa aatatataga aacgcataca 3660
catcattttg cgaattttac aacttctcaa cgtcaaggac tcacgtttga atcaaataaa 3720
gtgacagtga ccatttcttc agaagatgga gaattcttag tggataatat tgcgcttgtg 3780
gaagctcctc ttcctacaga tgaccaaaat tctgagggaa atacggcttc cagtacgaat 3840
agcgatacaa gtatgaacaa caatcaa 3867

<210> 4
<211> 1289
<212> PRT
<213> Bacillus thuringiensis

<400> 4
Met Ala Ile Leu Asn Glu Leu Tyr Pro Ser Val Pro Tyr Asn Val Leu
1               5                   10                  15

Ala Tyr Thr Pro Pro Ser Phe Leu Pro Asp Ala Gly Thr Gln Ala Thr
            20                  25                  30

Pro Ala Asp Leu Thr Ala Tyr Glu Gln Leu Leu Lys Asn Leu Glu Lys
        35                  40                  45

Gly Ile Asn Ala Gly Thr Tyr Ser Lys Ala Ile Ala Asp Val Leu Lys
    50                  55                  60

Gly Ile Phe Ile Asp Asp Thr Ile Asn Tyr Gln Thr Tyr Val Asn Ile
65                  70                  75                  80

Gly Leu Ser Leu Ile Thr Leu Ala Val Pro Glu Ile Gly Ile Phe Thr
                85                  90                  95

Pro Phe Ile Gly Leu Phe Phe Ala Ala Leu Asn Lys His Asp Ala Pro
            100                 105                 110

Pro Pro Pro Asn Ala Lys Asp Ile Phe Glu Ala Met Lys Pro Ala Ile
        115                 120                 125

Gln Glu Met Ile Asp Arg Thr Leu Thr Ala Asp Glu Gln Thr Phe Leu
    130                 135                 140

Asn Gly Glu Ile Ser Gly Leu Gln Asn Leu Ala Ala Arg Tyr Gln Ser
145                 150                 155                 160

Thr Met Asp Asp Ile Gln Ser His Gly Gly Phe Asn Lys Val Asp Ser
                165                 170                 175

Gly Leu Ile Lys Lys Phe Thr Asp Glu Val Leu Ser Leu Asn Ser Phe
            180                 185                 190

Tyr Thr Asp Arg Leu Pro Val Phe Ile Thr Asp Asn Thr Ala Asp Arg
        195                 200                 205

Thr Leu Leu Gly Leu Pro Tyr Tyr Ala Ile Leu Ala Ser Met His Leu
    210                 215                 220

Met Leu Leu Arg Asp Ile Ile Thr Lys Gly Pro Thr Trp Asp Ser Lys
225                 230                 235                 240

Ile Asn Phe Thr Pro Asp Ala Ile Asp Ser Phe Lys Thr Asp Ile Lys
                245                 250                 255

Asn Asn Ile Lys Leu Tyr Ser Lys Thr Ile Tyr Asp Val Phe Gln Lys
            260                 265                 270

Gly Leu Ala Ser Tyr Gly Thr Pro Ser Asp Leu Glu Ser Phe Ala Lys
        275                 280                 285

Lys Gln Lys Tyr Ile Glu Ile Met Thr Thr His Cys Leu Asp Phe Ala
    290                 295                 300

Arg Leu Phe Pro Thr Phe Asp Pro Asp Leu Tyr Pro Thr Gly Ser Gly
305                 310                 315                 320

Asp Ile Ser Leu Gln Lys Thr Arg Arg Ile Leu Ser Pro Phe Ile Pro
                325                 330                 335

Ile Arg Thr Ala Asp Gly Leu Thr Leu Asn Asn Thr Ser Ile Asp Thr
            340                 345                 350

Ser Asn Trp Pro Asn Tyr Glu Asn Gly Asn Gly Ala Phe Pro Asn Pro
        355                 360                 365

Lys Glu Arg Ile Leu Lys Gln Phe Lys Leu Tyr Pro Ser Trp Arg Ala
    370                 375                 380

Ala Gln Tyr Gly Gly Leu Leu Gln Pro Tyr Leu Trp Ala Ile Glu Val
385                 390                 395                 400

Gln Asp Ser Val Glu Thr Arg Leu Tyr Gly Gln Leu Pro Ala Val Asp
                405                 410                 415

Pro Gln Ala Gly Pro Asn Tyr Val Ser Ile Asp Ser Ser Asn Pro Ile
            420                 425                 430

Ile Gln Ile Asn Met Asp Thr Trp Lys Thr Pro Pro Gln Gly Ala Ser
        435                 440                 445

Gly Trp Asn Thr Asn Leu Met Arg Gly Ser Val Ser Gly Leu Ser Phe
    450                 455                 460

Leu Gln Arg Asp Gly Thr Arg Leu Ser Ala Gly Met Gly Gly Gly Phe
465                 470                 475                 480

Ala Asp Thr Ile Tyr Ser Leu Pro Ala Thr His Tyr Leu Ser Tyr Leu
                485                 490                 495

Tyr Gly Thr Pro Tyr Gln Thr Ser Asp Asn Tyr Ser Gly His Val Gly
            500                 505                 510

Ala Leu Val Gly Val Ser Thr Pro Gln Glu Ala Thr Leu Pro Asn Ile
        515                 520                 525

Ile Gly Gln Pro Asp Glu Gln Gly Asn Val Ser Thr Met Gly Phe Pro
    530                 535                 540

Phe Glu Lys Ala Ser Tyr Gly Gly Thr Val Val Lys Glu Trp Leu Asn
545                 550                 555                 560

Gly Ala Asn Ala Met Lys Leu Ser Pro Gly Gln Ser Ile Gly Ile Pro
                565                 570                 575

Ile Thr Asn Val Thr Ser Gly Glu Tyr Gln Ile Arg Cys Arg Tyr Ala
            580                 585                 590

Ser Asn Asp Asn Thr Asn Val Phe Phe Asn Val Asp Thr Gly Gly Ala
        595                 600                 605

Asn Pro Ile Phe Gln Gln Ile Asn Phe Ala Ser Thr Val Asp Asn Asn
    610                 615                 620

Thr Gly Val Gln Gly Ala Asn Gly Val Tyr Val Val Lys Ser Ile Ala
625                 630                 635                 640

Thr Thr Asp Asn Ser Phe Thr Val Lys Ile Pro Ala Lys Thr Ile Asn
                645                 650                 655

Val His Leu Thr Asn Gln Gly Ser Ser Asp Val Phe Leu Asp Arg Ile
            660                 665                 670

Glu Phe Val Pro Ile Leu Glu Ser Asn Thr Val Thr Ile Phe Asn Asn
        675                 680                 685

Ser Tyr Thr Thr Gly Ser Ala Asn Leu Ile Pro Ala Ile Ala Pro Leu
690                 695                 700

Trp Ser Thr Ser Ser Asp Lys Ala Leu Thr Gly Ser Met Ser Ile Thr
705                 710                 715                 720

Gly Arg Thr Thr Pro Asn Ser Asp Asp Ala Leu Leu Arg Phe Phe Lys
                725                 730                 735

Thr Asn Tyr Asp Thr Gln Thr Ile Pro Ile Pro Gly Ser Gly Lys Asp
            740                 745                 750

Phe Thr Asn Thr Leu Glu Ile Gln Asp Ile Val Ser Ile Asp Ile Phe
        755                 760                 765

Val Gly Ser Gly Leu His Gly Ser Asp Gly Ser Ile Lys Leu Asp Phe
    770                 775                 780

Thr Asn Asn Asn Ser Gly Ser Gly Gly Ser Pro Lys Ser Phe Thr Glu
785                 790                 795                 800

Gln Asn Asp Leu Glu Asn Ile Thr Thr Gln Val Asn Ala Leu Phe Thr
                805                 810                 815

Ser Asn Thr Gln Asp Ala Leu Ala Thr Asp Val Ser Asp His Asp Ile
            820                 825                 830

Glu Glu Val Val Leu Lys Val Asp Ala Leu Ser Asp Glu Val Phe Gly
        835                 840                 845

Lys Glu Lys Lys Thr Leu Arg Lys Phe Val Asn Gln Ala Lys Arg Leu
    850                 855                 860

Ser Lys Ala Arg Asn Leu Leu Val Gly Gly Asn Phe Asp Asn Leu Asp
865                 870                 875                 880

Ala Trp Tyr Arg Gly Arg Asn Val Val Asn Val Ser Asn His Glu Leu
                885                 890                 895

Leu Lys Ser Asp His Val Leu Leu Pro Pro Pro Gly Leu Ser Pro Ser
            900                 905                 910

Tyr Ile Phe Gln Lys Val Glu Glu Ser Lys Leu Lys Arg Asn Thr Arg
        915                 920                 925

Tyr Thr Val Ser Gly Phe Ile Ala His Ala Thr Asp Leu Glu Ile Val
    930                 935                 940

Val Ser Arg Tyr Gly Gln Glu Ile Lys Lys Val Val Gln Val Pro Tyr
945                 950                 955                 960

Gly Glu Ala Phe Pro Leu Thr Ser Ser Gly Pro Val Cys Cys Ile Pro
                965                 970                 975

His Ser Thr Ser Asn Gly Thr Leu Gly Asn Pro His Phe Phe Ser Tyr
            980                 985                 990

Ser Ile Asp Val Gly Ala Leu Asp Val Asp Thr Asn Pro Gly Ile Glu
        995                 1000                1005

Phe Gly Leu Arg Ile Val Asn Pro Thr Gly Met Ala Arg Val Ser Asn
    1010                1015                1020

Leu Glu Ile Arg Glu Asp Arg Pro Leu Ala Ala Asn Glu Ile Arg Gln
1025                1030                1035                1040

Val Gln Arg Val Ala Arg Asn Trp Arg Thr Glu Tyr Glu Lys Glu Arg
                1045                1050                1055

Ala Glu Val Thr Ser Leu Ile Gln Pro Val Ile Asn Arg Ile Asn Gly
            1060                1065                1070

Leu Tyr Asp Asn Gly Asn Trp Asn Gly Ser Ile Arg Ser Asp Ile Ser
        1075                1080                1085

Tyr Gln Asn Ile Asp Ala Ile Val Leu Pro Thr Leu Pro Lys Leu Arg
    1090                1095                1100

His Trp Phe Met Ser Asp Arg Phe Ser Glu Gln Gly Asp Ile Met Ala
1105                1110                1115                1120

Lys Phe Gln Gly Ala Leu Asn Arg Ala Tyr Ala Gln Leu Glu Gln Asn
                1125                1130                1135

Thr Leu Leu His Asn Gly His Phe Thr Lys Asp Ala Ala Asn Trp Thr
            1140                1145                1150

Val Glu Gly Asp Ala His Gln Val Val Leu Glu Asp Gly Lys Arg Val
        1155                1160                1165

Leu Arg Leu Pro Asp Trp Ser Ser Ser Val Ser Gln Thr Ile Glu Ile
    1170                1175                1180

Glu Asn Phe Asp Pro Asp Lys Glu Tyr Gln Leu Val Phe His Gly Gln
1185                1190                1195                1200

Gly Glu Gly Thr Val Thr Leu Glu His Gly Glu Glu Thr Lys Tyr Ile
                1205                1210                1215

Glu Thr His Thr His His Phe Ala Asn Phe Thr Thr Ser Gln Arg Gln
            1220                1225                1230

Gly Leu Thr Phe Glu Ser Asn Lys Val Thr Val Thr Ile Ser Ser Glu
        1235                1240                1245

Asp Gly Glu Phe Leu Val Asp Asn Ile Ala Leu Val Glu Ala Pro Leu
    1250                1255                1260

Pro Thr Asp Asp Gln Asn Ser Glu Gly Asn Thr Ala Ser Ser Thr Asn
1265                1270                1275                1280

Ser Asp Thr Ser Met Asn Asn Asn Gln
                1285

<210> 5
<211> 3771
<212> DNA
<213> Bacillus thuringiensis

atggctacac ttaatgaagt atatcctgtg aattataatg tattatcttc tgatgctttt 60
caacaattag atacaacagg ttttaaaagt aaatatgatg aaatgataaa agcattcgaa 120
aaaaaatgga aaaaaggggc aaaaggaaaa gaccttttag atgttgcatg gacttatata 180
actacaggag aaattgaccc tttaaatgta attaaaggtg ttttatctgt attaacttta 240
attcctgaag ttggtactgt ggcctctgca gcaagtacta ttgtaagttt tatttggcct 300
aaaatatttg gagataaacc aaatgcaaaa aatatatttg aagagctcaa gcctcaaatt 360
gaagcattaa ttcaacaaga tataacaaac tatcaagatg caattaatca aaaaaaattt 420
gacagtcttc agaaaacaat taatctatat acagtagcta tagataacaa tgattacgta 480
acagcaaaaa cgcaactcga aaatctaaat tctatactta cctcagatat ctccatattt 540
attccagaag gatatgaaac tggaggttta ccttattatg ctatggttgc taatgctcat 600
atattattgt taagagacgc tatagttaat gcagagaaat taggctttag tgataaagaa 660
gtagacacac ataaaaaata tatcaaaatg acaatacaca atcatactga agcagtaata 720
aaagcattct taaatggact tgacaaattt aagagtttag atgtaaatag ctataataaa 780
aaagcaaatt atattaaagg tatgacagaa atggttcttg atctagttgc tctatggcca 840
actttcgatc cagatcatta tcaaaaagaa gtagaaattg aatttacaag aactatttct 900
tctccaattt accaacctgt acctaaaaac atgcaaaata cctctagctc tattgtacct 960
agcgatctat ttcactatca aggagatctt gtaaaattag aattttctac aagaacggac 1020
aacgatggtc ttgcaaaaat ttttactggt attcgaaaca cattctacaa atcgcctaat 1080
actcatgaaa cataccatgt agattttagt tataataccc aatctagtgg taatatttca 1140
agaggctctt caaatccgat tccaattgat cttaataatc ccattatttc aacttgtatt 1200
agaaattcat tttataaggc aatagcggga tcttctgttt tagttaattt taaagatggc 1260
actcaagggt atgcatttgc ccaagcacca acaggaggtg cctgggacca ttcttttatt 1320
gaatctgatg gtgccccaga agggcataaa ttaaactata tttatacttc tccaggtgat 1380
acattaagag atttcatcaa tgtatatact cttataagta ctccaactat aaatgaacta 1440

tcaacagaaa aaatcaaagg ctttcctgcg gaaaaaggat atatcaaaaa tcaagggatc 1500
atgaaatatt acggtaaacc agaatatatt aatggagctc aaccagttaa tctggaaaac 1560
cagcaaacat taatattcga atttcatgct tcaaaaacag ctcaatatac cattcgtata 1620
cgttatgcca gtacccaagg aacaaaaggt tattttcgtt tagataatca ggaactgcaa 1680
acgcttaata tacctacttc acacaacggt tatgtaaccg gtaatattgg tgaaaattat 1740
gatttatata caataggttc atatacaatt acagaaggta accatactct tcaaatccaa 1800
cataatgata aaaatggaat ggttttagat cgtattgaat ttgttcctaa agattcactt 1860
caagattcac ctcaagattc acctccagaa gttcacgaat caacaattat ttttgataaa 1920
tcatctccaa ctatatggtc ttctaacaaa cactcatata gccatataca tttagaagga 1980
tcatatacaa gtcagggaag ttatccacac aatttattaa ttaatttatt tcatcctaca 2040
gaccctaaca gaaatcatac tattcatgtt aacaatggtg atatgaatgt tgattatgga 2100
aaagattctg tagccgatgg gttaaatttt aataaaataa ctgctacgat accaagtgat 2160
gcttggtata gcggtactat tacttctatg cacttattta atgataataa ttttaaaaca 2220
ataactccta aatttgaact ttctaatgaa ttagaaaaca tcacaactca agtaaatgct 2280
ttattcgcat ctagtgcaca agatactctc gcaagtaatg taagtgatta ctggattgaa 2340
caggtcgtta tgaaagtcga tgccttatca gatgaagtat ttggaaaaga gaaaaaagca 2400
ttacgtaaat tggtaaatca agcaaaacgt ctcagtaaaa tacgaaatct tctcataggt 2460
ggtaattttg acaatttagt cgcttggtat atgggaaaag atgtagtaaa agaatcggat 2520
catgaattat ttaaaagtga tcatgtctta ctacctcccc caacattcca tccttcttat 2580
attttccaaa aggtggaaga atcaaaacta aaaccaaata cacgttatac tatttctggt 2640
tttatcgcac atggagaaga tgtagagctt gttgtctctc gttatgggca agaaatacaa 2700
aaagtgatgc aagtgccata tgaagaagca cttcctctta catctgaatc taattctagt 2760
tgttgtgttc caaatttaaa tataaatgaa acactagctg atccacattt ctttagttat 2820
agcatcgatg ttggttctct ggaaatggaa gcgaatcctg gtattgaatt tggtctccgt 2880
attgtcaaac caacaggtat ggcacgtgta agtaatttag aaattcgaga agaccgtcca 2940
ttaacagcaa aagaaattcg tcaagtacaa cgtgcagcaa gagattggaa acaaaactat 3000
gaacaagaac gaacagagat cacagctata attcaacctg ttcttaatca aattaatgcg 3060
ttatacgaaa atgaagattg gaatggttct attcgttcaa atgtttccta tcatgatcta 3120
gagcaaatta tgcttcctac tttattaaaa actgaggaaa taaattgtaa ttatgatcat 3180
ccagcttttt tattaaaagt atatcattgg tttatgacag atcgtatagg agaacatggt 3240
actattttag cacgtttcca agaagcatta gatcgtgcat atacacaatt agaaagtcgt 3300
aatctcctgc ataacggtca ttttacaact gatacagcga attggacaat agaaggagat 3360
gcccatcata caatcttaga agatggtaga cgtgtgttac gtttaccaga ttggtcttct 3420
aatgcaactc aaacaattga aattgaagat tttgacttag atcaagaata ccaattgctc 3480
attcatgcaa aaggaaaagg ttccattact ttacaacatg gagaagaaaa cgaatatgtg 3540
gaaacacata ctcatcatac aaatgatttt ataacatccc aaaatattcc tttcactttt 3600
aaaggaaatc aaattgaagt ccatattact tcagaagatg gagagttttt aatcgatcac 3660
attacagtaa tagaagtttc taaaacagac acaaatacaa atattattga aaattcacca 3720
atcaatacaa gtatgaatag taatgtaaga gtagatatac caagaagtct c 3771

<210> 6
<211> 1257
<212> PRT
<213> Bacillus thuringiensis

Met Ala Thr Leu Asn Glu Val Tyr Pro Val Asn Tyr Asn Val Leu Ser
1               5                   10                  15

Ser Asp Ala Phe Gln Gln Leu Asp Thr Thr Gly Phe Lys Ser Lys Tyr
            20                  25                  30

Asp Glu Met Ile Lys Ala Phe Glu Lys Lys Trp Lys Lys Gly Ala Lys
        35                  40                  45

Gly Lys Asp Leu Leu Asp Val Ala Trp Thr Tyr Ile Thr Thr Gly Glu
    50                  55                  60

Ile Asp Pro Leu Asn Val Ile Lys Gly Val Leu Ser Val Leu Thr Leu
65                  70                  75                  80

Ile Pro Glu Val Gly Thr Val Ala Ser Ala Ala Ser Thr Ile Val Ser
                85                  90                  95

Phe Ile Trp Pro Lys Ile Phe Gly Asp Lys Pro Asn Ala Lys Asn Ile
            100                 105                 110

Phe Glu Glu Leu Lys Pro Gln Ile Glu Ala Leu Ile Gln Gln Asp Ile
        115                 120                 125

Thr Asn Tyr Gln Asp Ala Ile Asn Gln Lys Lys Phe Asp Ser Leu Gln
    130                 135                 140

Lys Thr Ile Asn Leu Tyr Thr Val Ala Ile Asp Asn Asn Asp Tyr Val
145                 150                 155                 160

Thr Ala Lys Thr Gln Leu Glu Asn Leu Asn Ser Ile Leu Thr Ser Asp
                165                 170                 175

Ile Ser Ile Phe Ile Pro Glu Gly Tyr Glu Thr Gly Gly Leu Pro Tyr
            180                 185                 190

Tyr Ala Met Val Ala Asn Ala His Ile Leu Leu Leu Arg Asp Ala Ile
        195                 200                 205

Val Asn Ala Glu Lys Leu Gly Phe Ser Asp Lys Glu Val Asp Thr His
    210                 215                 220

Lys Lys Tyr Ile Lys Met Thr Ile His Asn His Thr Glu Ala Val Ile
225                 230                 235                 240

Lys Ala Phe Leu Asn Gly Leu Asp Lys Phe Lys Ser Leu Asp Val Asn
                245                 250                 255

Ser Tyr Asn Lys Lys Ala Asn Tyr Ile Lys Gly Met Thr Glu Met Val
            260                 265                 270

Leu Asp Leu Val Ala Leu Trp Pro Thr Phe Asp Pro Asp His Tyr Gln
        275                 280                 285

Lys Glu Val Glu Ile Glu Phe Thr Arg Thr Ile Ser Ser Pro Ile Tyr
    290                 295                 300

Gln Pro Val Pro Lys Asn Met Gln Asn Thr Ser Ser Ser Ile Val Pro
305                 310                 315                 320

Ser Asp Leu Phe His Tyr Gln Gly Asp Leu Val Lys Leu Glu Phe Ser
                325                 330                 335

Thr Arg Thr Asp Asn Asp Gly Leu Ala Lys Ile Phe Thr Gly Ile Arg
            340                 345                 350

Asn Thr Phe Tyr Lys Ser Pro Asn Thr His Glu Thr Tyr His Val Asp
        355                 360                 365

Phe Ser Tyr Asn Thr Gln Ser Ser Gly Asn Ile Ser Arg Gly Ser Ser
    370                 375                 380

Asn Pro Ile Pro Ile Asp Leu Asn Asn Pro Ile Ile Ser Thr Cys Ile
385                 390                 395                 400

Arg Asn Ser Phe Tyr Lys Ala Ile Ala Gly Ser Ser Val Leu Val Asn
                405                 410                 415

Phe Lys Asp Gly Thr Gln Gly Tyr Ala Phe Ala Gln Ala Pro Thr Gly
            420                 425                 430

Gly Ala Trp Asp His Ser Phe Ile Glu Ser Asp Gly Ala Pro Glu Gly
        435                 440                 445

His Lys Leu Asn Tyr Ile Tyr Thr Ser Pro Gly Asp Thr Leu Arg Asp
    450                 455                 460

Phe Ile Asn Val Tyr Thr Leu Ile Ser Thr Pro Thr Ile Asn Glu Leu
465                 470                 475                 480

Ser Thr Glu Lys Ile Lys Gly Phe Pro Ala Glu Lys Gly Tyr Ile Lys
                485                 490                 495

Asn Gln Gly Ile Met Lys Tyr Tyr Gly Lys Pro Glu Tyr Ile Asn Gly
            500                 505                 510

Ala Gln Pro Val Asn Leu Glu Asn Gln Gln Thr Leu Ile Phe Glu Phe
        515                 520                 525

His Ala Ser Lys Thr Ala Gln Tyr Thr Ile Arg Ile Arg Tyr Ala Ser
    530                 535                 540

Thr Gln Gly Thr Lys Gly Tyr Phe Arg Leu Asp Asn Gln Glu Leu Gln
545                 550                 555                 560

Thr Leu Asn Ile Pro Thr Ser His Asn Gly Tyr Val Thr Gly Asn Ile
                565                 570                 575

Gly Glu Asn Tyr Asp Leu Tyr Thr Ile Gly Ser Tyr Thr Ile Thr Glu
            580                 585                 590

Gly Asn His Thr Leu Gln Ile Gln His Asn Asp Lys Asn Gly Met Val
        595                 600                 605

Leu Asp Arg Ile Glu Phe Val Pro Lys Asp Ser Leu Gln Asp Ser Pro
    610                 615                 620

Gln Asp Ser Pro Pro Glu Val His Glu Ser Thr Ile Ile Phe Asp Lys
625                 630                 635                 640

Ser Ser Pro Thr Ile Trp Ser Ser Asn Lys His Ser Tyr Ser His Ile
                645                 650                 655

His Leu Glu Gly Ser Tyr Thr Ser Gln Gly Ser Tyr Pro His Asn Leu
            660                 665                 670

Leu Ile Asn Leu Phe His Pro Thr Asp Pro Asn Arg Asn His Thr Ile
        675                 680                 685

His Val Asn Asn Gly Asp Met Asn Val Asp Tyr Gly Lys Asp Ser Val
    690                 695                 700

Ala Asp Gly Leu Asn Phe Asn Lys Ile Thr Ala Thr Ile Pro Ser Asp
705                 710                 715                 720

Ala Trp Tyr Ser Gly Thr Ile Thr Ser Met His Leu Phe Asn Asp Asn
                725                 730                 735

Asn Phe Lys Thr Ile Thr Pro Lys Phe Glu Leu Ser Asn Glu Leu Glu
            740                 745                 750

Asn Ile Thr Thr Gln Val Asn Ala Leu Phe Ala Ser Ser Ala Gln Asp
        755                 760                 765

Thr Leu Ala Ser Asn Val Ser Asp Tyr Trp Ile Glu Gln Val Val Met
    770                 775                 780

Lys Val Asp Ala Leu Ser Asp Glu Val Phe Gly Lys Glu Lys Lys Ala
785                 790                 795                 800

Leu Arg Lys Leu Val Asn Gln Ala Lys Arg Leu Ser Lys Ile Arg Asn
                805                 810                 815

Leu Leu Ile Gly Gly Asn Phe Asp Asn Leu Val Ala Trp Tyr Met Gly
            820                 825                 830

Lys Asp Val Val Lys Glu Ser Asp His Glu Leu Phe Lys Ser Asp His
        835                 840                 845

Val Leu Leu Pro Pro Pro Thr Phe His Pro Ser Tyr Ile Phe Gln Lys
    850                 855                 860

Val Glu Glu Ser Lys Leu Lys Pro Asn Thr Arg Tyr Thr Ile Ser Gly
865                 870                 875                 880

Phe Ile Ala His Gly Glu Asp Val Glu Leu Val Val Ser Arg Tyr Gly
                885                 890                 895

Gln Glu Ile Gln Lys Val Met Gln Val Pro Tyr Glu Glu Ala Leu Pro
            900                 905                 910

Leu Thr Ser Glu Ser Asn Ser Ser Cys Cys Val Pro Asn Leu Asn Ile
        915                 920                 925

Asn Glu Thr Leu Ala Asp Pro His Phe Phe Ser Tyr Ser Ile Asp Val
    930                 935                 940

Gly Ser Leu Glu Met Glu Ala Asn Pro Gly Ile Glu Phe Gly Leu Arg
945                 950                 955                 960

Ile Val Lys Pro Thr Gly Met Ala Arg Val Ser Asn Leu Glu Ile Arg
                965                 970                 975

Glu Asp Arg Pro Leu Thr Ala Lys Glu Ile Arg Gln Val Gln Arg Ala
            980                 985                 990

Ala Arg Asp Trp Lys Gln Asn Tyr Glu Gln Glu Arg Thr Glu Ile Thr
        995                 1000                1005

Ala Ile Ile Gln Pro Val Leu Asn Gln Ile Asn Ala Leu Tyr Glu Asn
    1010                1015                1020

Glu Asp Trp Asn Gly Ser Ile Arg Ser Asn Val Ser Tyr His Asp Leu
1025                1030                1035                1040

Glu Gln Ile Met Leu Pro Thr Leu Leu Lys Thr Glu Glu Ile Asn Cys
            1045                1050                1055

Asn Tyr Asp His Pro Ala Phe Leu Leu Lys Val Tyr His Trp Phe Met
        1060                1065                1070

Thr Asp Arg Ile Gly Glu His Gly Thr Ile Leu Ala Arg Phe Gln Glu
    1075                1080                1085

Ala Leu Asp Arg Ala Tyr Thr Gln Leu Glu Ser Arg Asn Leu Leu His
    1090                1095                1100

Asn Gly His Phe Thr Thr Asp Thr Ala Asn Trp Thr Ile Glu Gly Asp
1105                1110                1115                1120

Ala His His Thr Ile Leu Glu Asp Gly Arg Arg Val Leu Arg Leu Pro
            1125                1130                1135

Asp Trp Ser Ser Asn Ala Thr Gln Thr Ile Glu Ile Glu Asp Phe Asp
        1140                1145                1150

Leu Asp Gln Glu Tyr Gln Leu Leu Ile His Ala Lys Gly Lys Gly Ser
    1155                1160                1165

Ile Thr Leu Gln His Gly Glu Glu Asn Glu Tyr Val Glu Thr His Thr
    1170                1175                1180

His His Thr Asn Asp Phe Ile Thr Ser Gln Asn Ile Pro Phe Thr Phe
1185                1190                1195                1200

Lys Gly Asn Gln Ile Glu Val His Ile Thr Ser Glu Asp Gly Glu Phe
            1205                1210                1215

Leu Ile Asp His Ile Thr Val Ile Glu Val Ser Lys Thr Asp Thr Asn
        1220                1225                1230

Thr Asn Ile Ile Glu Asn Ser Pro Ile Asn Thr Ser Met Asn Ser Asn
    1235                1240                1245

Val Arg Val Asp Ile Pro Arg Ser Leu
    1250                1255


<210> 7
<211> 1425
<212> DNA
<213> Bacillus thuringiensis

atgattattg atagtaaaac gactttacct agacattcac ttattcatac aattaaatta 60
aattctaata agaaatatgg tcctggtgat atgactaatg gaaatcaatt tattatttca 120
aaacaagaat gggctacgat tggagcatat attcagactg gattaggttt accagtaaat 180
gaacaacaat taagaacaca tgttaattta agtcaggata tatcaatacc tagtgatttt 240
tctcaattat atgatgttta ttgttctgat aaaacttcag cagaatggtg gaataaaaat 300
ttatatcctt taattattaa atctgctaat gatattgctt catatggttt taaagttgct 360
ggtgatcctt ctattaagaa agatggatat tttaaaaaat tgcaagatga attagataat 420
attgttgata ataattccga tgatgatgca atagctaaag ctattaaaga ttttaaagcg 480
cgatgtggta ttttaattaa agaagctaaa caatatgaag aagctgcaaa aaatattgta 540
acatctttag atcaattttt acatggtgat cagaaaaaat tagaaggtgt tatcaatatt 600
caaaaacgtt taaaagaagt tcaaacagct cttaatcaag cccatgggga aagtagtcca 660
gctcataaag agttattaga aaaagtaaaa aatttaaaaa caacattaga aaggactatt 720
aaagctgaac aagatttaga gaaaaaagta gaatatagtt ttctattagg accattgtta 780
ggatttgttg tttatgaaat tcttgaaaat actgctgttc agcatataaa aaatcaaatt 840
gatgagataa agaaacaatt agattctgct cagcatgatt tggatagaga tgttaaaatt 900
ataggaatgt taaatagtat taatacagat attgataatt tatatagtca aggacaagaa 960
gcaattaaag ttttccaaaa gttacaaggt atttgggcta ctattggagc tcaaatagaa 1020
aatcttagaa caacgtcgtt acaagaagtt caagattctg atgatgctga tgagatacaa 1080
attgaacttg aggacgcttc tgatgcttgg ttagttgtgg ctcaagaagc tcgtgatttt 1140
acactaaatg cttattcaac taatagtaga caaaatttac cgattaatgt tatatcagat 1200
tcatgtaatt gttcaacaac aaatatgaca tcaaatcaat acagtaatcc aacaacaaat 1260
atgacatcaa atcaatatat gatttcacat gaatatacaa gtttaccaaa taattttatg 1320
ttatcaagaa atagtaattt agaatataaa tgtcctgaaa ataattttat gatatattgg 1380
tataataatt cggattggta taataattcg gattggtata ataat 1425

<210> 8
<211> 475
<212> PRT
<213> Bacillus thuringiensis

Met Ile Ile Asp Ser Lys Thr Thr Leu Pro Arg His Ser Leu Ile His
1               5                   10                  15

Thr Ile Lys Leu Asn Ser Asn Lys Lys Tyr Gly Pro Gly Asp Met Thr
            20                  25                  30

Asn Gly Asn Gln Phe Ile Ile Ser Lys Gln Glu Trp Ala Thr Ile Gly
        35                  40                  45

Ala Tyr Ile Gln Thr Gly Leu Gly Leu Pro Val Asn Glu Gln Gln Leu
    50                  55                  60

Arg Thr His Val Asn Leu Ser Gln Asp Ile Ser Ile Pro Ser Asp Phe
65                  70                  75                  80

Ser Gln Leu Tyr Asp Val Tyr Cys Ser Asp Lys Thr Ser Ala Glu Trp
                85                  90                  95

Trp Asn Lys Asn Leu Tyr Pro Leu Ile Ile Lys Ser Ala Asn Asp Ile
            100                 105                 110

Ala Ser Tyr Gly Phe Lys Val Ala Gly Asp Pro Ser Ile Lys Lys Asp
        115                 120                 125

Gly Tyr Phe Lys Lys Leu Gln Asp Glu Leu Asp Asn Ile Val Asp Asn
    130                 135                 140

Asn Ser Asp Asp Asp Ala Ile Ala Lys Ala Ile Lys Asp Phe Lys Ala
145                 150                 155                 160

Arg Cys Gly Ile Leu Ile Lys Glu Ala Lys Gln Tyr Glu Glu Ala Ala
                165                 170                 175

Lys Asn Ile Val Thr Ser Leu Asp Gln Phe Leu His Gly Asp Gln Lys
            180                 185                 190

Lys Leu Glu Gly Val Ile Asn Ile Gln Lys Arg Leu Lys Glu Val Gln
        195                 200                 205

Thr Ala Leu Asn Gln Ala His Gly Glu Ser Ser Pro Ala His Lys Glu
    210                 215                 220

Leu Leu Glu Lys Val Lys Asn Leu Lys Thr Thr Leu Glu Arg Thr Ile
225                 230                 235                 240

Lys Ala Glu Gln Asp Leu Glu Lys Lys Val Glu Tyr Ser Phe Leu Leu
                245                 250                 255

Gly Pro Leu Leu Gly Phe Val Val Tyr Glu Ile Leu Glu Asn Thr Ala
            260                 265                 270

Val Gln His Ile Lys Asn Gln Ile Asp Glu Ile Lys Lys Gln Leu Asp
        275                 280                 285

Ser Ala Gln His Asp Leu Asp Arg Asp Val Lys Ile Ile Gly Met Leu
    290                 295                 300

Asn Ser Ile Asn Thr Asp Ile Asp Asn Leu Tyr Ser Gln Gly Gln Glu
305                 310                 315                 320

Ala Ile Lys Val Phe Gln Lys Leu Gln Gly Ile Trp Ala Thr Ile Gly
                325                 330                 335

Ala Gln Ile Glu Asn Leu Arg Thr Thr Ser Leu Gln Glu Val Gln Asp
            340                 345                 350

Ser Asp Asp Ala Asp Glu Ile Gln Ile Glu Leu Glu Asp Ala Ser Asp
        355                 360                 365

Ala Trp Leu Val Val Ala Gln Glu Ala Arg Asp Phe Thr Leu Asn Ala
    370                 375                 380

Tyr Ser Thr Asn Ser Arg Gln Asn Leu Pro Ile Asn Val Ile Ser Asp
385                 390                 395                 400

Ser Cys Asn Cys Ser Thr Thr Asn Met Thr Ser Asn Gln Tyr Ser Asn
                405                 410                 415

Pro Thr Thr Asn Met Thr Ser Asn Gln Tyr Met Ile Ser His Glu Tyr
            420                 425                 430

Thr Ser Leu Pro Asn Asn Phe Met Leu Ser Arg Asn Ser Asn Leu Glu
        435                 440                 445

Tyr Lys Cys Pro Glu Asn Asn Phe Met Ile Tyr Trp Tyr Asn Asn Ser
    450                 455                 460

Asp Trp Tyr Asn Asn Ser Asp Trp Tyr Asn Asn
465                 470                 475

<210> 9
<211> 1185
<212> DNA
<213> Bacillus thuringiensis

atgattttag ggaatggaaa gactttacca aagcatataa gattagctca tatttttgca 60
acacagaatt cttcagctaa gaaagacaat cctcttggac cagaggggat ggttactaaa 120
gacggtttta taatctctaa ggaagaatgg gcatttgtgc aggcctatgt gactacaggc 180
actggtttac ctatcaatga cgatgagatg cgtagacatg ttgggttacc atcacgcatt 240
caaattcctg atgattttaa tcaattatat aaggtttata atgaagataa acatttatgc 300
agttggtgga atggtttctt gtttccatta gttcttaaaa cagctaatga tatttccgct 360
tacggattta aatgtgctgg aaagggtgcc actaaaggat attatgaggt catgcaagac 420
gatgtagaaa atatttcaga taatggttat gataaagttg cacaagaaaa agcacataag 480
gatctgcagg cgcgttgtaa aatccttatt aaggaggctg atcaatataa agctgcagcg 540
gatgatgttt caaaacattt aaacacattt cttaaaggcg gtcaagattc agatggcaat 600
gatgttattg gcgtagaggc tgttcaagta caactagcac aagtaaaaga taatcttgat 660
ggcctatatg gcgacaaaag cccaagacat gaagagttac taaagaaagt agacgacctg 720
aaaaaagagt tggaagctgc tattaaagca gagaatgaat tagaaaagaa agtgaaaatg 780
agttttgctt taggaccatt acttggattt gttgtatatg aaatcttaga gctaactgcg 840
gtcaaaagta tacacaagaa agttgaggca ctacaagccg agcttgacac tgctaatgat 900

gaactcgaca gagatgtaaa aatcttagga atgatgaata gcattgacac tgatattgac 960
aacatgttag agcaaggtga gcaagctctt gttgtattta gaaaaattgc aggcatttgg 1020
agtgttataa gtcttaatat cggcaatctt cgagaaacat ctttaaaaga gatagaagaa 1080
gaaaatgatg acgatgcact gtatattgag cttggtgatg ccgctggtca atggaaagag 1140
atagccgagg aggcacaatc ctttgtacta aatgcttata ctcct 1185

<210> 10
<211> 395
<212> PRT
<213> Bacillus thuringiensis

Met Ile Leu Gly Asn Gly Lys Thr Leu Pro Lys His Ile Arg Leu Ala
1               5                   10                  15

His Ile Phe Ala Thr Gln Asn Ser Ser Ala Lys Lys Asp Asn Pro Leu
            20                  25                  30

Gly Pro Glu Gly Met Val Thr Lys Asp Gly Phe Ile Ile Ser Lys Glu
        35                  40                  45

Glu Trp Ala Phe Val Gln Ala Tyr Val Thr Thr Gly Thr Gly Leu Pro
    50                  55                  60

Ile Asn Asp Asp Glu Met Arg Arg His Val Gly Leu Pro Ser Arg Ile
65                  70                  75                  80

Gln Ile Pro Asp Asp Phe Asn Gln Leu Tyr Lys Val Tyr Asn Glu Asp
                85                  90                  95

Lys His Leu Cys Ser Trp Trp Asn Gly Phe Leu Phe Pro Leu Val Leu
            100                 105                 110

Lys Thr Ala Asn Asp Ile Ser Ala Tyr Gly Phe Lys Cys Ala Gly Lys
        115                 120                 125

Gly Ala Thr Lys Gly Tyr Tyr Glu Val Met Gln Asp Asp Val Glu Asn
    130                 135                 140

Ile Ser Asp Asn Gly Tyr Asp Lys Val Ala Gln Glu Lys Ala His Lys
145                 150                 155                 160

Asp Leu Gln Ala Arg Cys Lys Ile Leu Ile Lys Glu Ala Asp Gln Tyr
                165                 170                 175

Lys Ala Ala Ala Asp Asp Val Ser Lys His Leu Asn Thr Phe Leu Lys
            180                 185                 190

Gly Gly Gln Asp Ser Asp Gly Asn Asp Val Ile Gly Val Glu Ala Val
        195                 200                 205

Gln Val Gln Leu Ala Gln Val Lys Asp Asn Leu Asp Gly Leu Tyr Gly
    210                 215                 220

Asp Lys Ser Pro Arg His Glu Glu Leu Leu Lys Lys Val Asp Asp Leu
225                 230                 235                 240

Lys Lys Glu Leu Glu Ala Ala Ile Lys Ala Glu Asn Glu Leu Glu Lys
                245                 250                 255

Lys Val Lys Met Ser Phe Ala Leu Gly Pro Leu Leu Gly Phe Val Val
            260                 265                 270

Tyr Glu Ile Leu Glu Leu Thr Ala Val Lys Ser Ile His Lys Lys Val
        275                 280                 285

Glu Ala Leu Gln Ala Glu Leu Asp Thr Ala Asn Asp Glu Leu Asp Arg
    290                 295                 300

Asp Val Lys Ile Leu Gly Met Met Asn Ser Ile Asp Thr Asp Ile Asp
305                 310                 315                 320

Asn Met Leu Glu Gln Gly Glu Gln Ala Leu Val Val Phe Arg Lys Ile
                325                 330                 335

Ala Gly Ile Trp Ser Val Ile Ser Leu Asn Ile Gly Asn Leu Arg Glu
            340                 345                 350

Thr Ser Leu Lys Glu Ile Glu Glu Glu Asn Asp Asp Asp Ala Leu Tyr
        355                 360                 365

Ile Glu Leu Gly Asp Ala Ala Gly Gln Trp Lys Glu Ile Ala Glu Glu
    370                 375                 380

Ala Gln Ser Phe Val Leu Asn Ala Tyr Thr Pro
385                 390                 395

## Claims

1. A Bacillus thuringiensis isolate active against nematodes, selected from strains PS167P; PS158D5; PS169E; PS177F1; PS177G; PS204G4; and PS204G6.

2. DNA comprising
(a) Sequence 1 or a fragment thereof which codes for a nematicidal toxin;
(b) Sequence 3 or a fragment thereof which codes for a nematicidal toxin;
(c) Sequence 5 or a fragment thereof which codes for a nematicidal toxin;
(d) Sequence 7 or a fragment thereof which codes for a nematicidal toxin;
(e) Sequence 9 or a fragment thereof which codes for a nematicidal toxin;
(f) the gene obtained from the nematicidally-active Bacillus thuringiensis isolate designated B.t. PS63B, having by restriction fragment length polymorphism analysis a 4.4 kb XbaI hybridising band, an N-terminal amino acid sequence that includes QLQAQPLIPYNVLA, and an internal amino acid sequence that includes VQRILDEKLSFQLIK, or a fragment thereof which codes for a nematicidal toxin;
(g) the gene obtained from the nematicidally-active Bacillus thuringiensis isolate Bacillus thuringiensis designated B.t. PS 17d, having by restriction fragment length polymorphism analysis a 5.0 kb EcoRI hybridising band, or a fragment thereof which codes for a nematicidal toxin;
(h) the gene obtained from the nematicidally-active Bacillus thuringiensis isolate Bacillus thuringiensis designated B.t. PS 17e, having by restriction fragment length polymorphism analysis a 1.8 kb EcoRI hybridising band, or a fragment thereof which codes for a nematicidal toxin; and
(i) a gene obtained from an isolate according to claim 1.

3. DNA encoding a nematicidal toxin having Sequence 2, Sequence 4, Sequence 6, Sequence 8 or Sequence 10.

4. A recombinant DNA transfer vector comprising a gene selected from the genes designated B.t. PS33F2, B.t. PS63B, B.t. PS52A1, B.t. PS69D1, B.t. PS17a, B.t. PS17b, B.t. PS17d, and B.t. PS17e.

5. A vector according to claim 4, selected from pMYC2316, pMYC2321, pMYC2317, pMYC1627, pMYC1628, pMYC2309, and pMYC2311.

6. A toxin active against nematodes, having an amino acid sequence as shown in any of Sequences 2, 4, 6, 8 and 10, or any part of any said Sequence which retains substantially all of the nematicidal activity of said Sequence.

7. A host cell transformed to express a Bacillus thuringiensis nematicidal toxin according to claim 6.

8. A host according to claim 7, which is selected from
(a) Escherichia coli (NM522)(pMYC2316), having the identifying nematicidal characteristics of NRRL B-18785;
(b) Escherichia coli (NM522)(pMYC2321), having the identifying nematicidal characteristics of NRRL B-18770;
(c) Escherichia coli (NM522)(pMYC2317), having the identifying nematicidal characteristics of NRRL B-18816;
(d) Escherichia coli (NM522)(pMYC 1627), having the identifying characteristics of NRRL B-18651;
(e) Escherichia coli (NM522)(pMYC1628), having the identifying characteristics of NRRL B-18652;
(f) Escherichia coli (NM522)(pMYC2311); and
(g) Escherichia coli (NM522) (pMYC2309).

9. A substantially intact recombinant cell comprising at least one intracellular nematicidal toxin, wherein said toxin is a result of expression of a Bacillus thuringiensis toxin gene which codes for a 6-endotoxin polypeptide toxin encoded by DNA according to claim 2.

10. A plant comprising DNA according to claim 2, as obtained by transforming the DNA into a plant or other host cell.

11. A process for controlling nematodes, which comprises contacting the nematodes with a toxin encoded by DNA according to claim 2, wherein the DNA has been transformed into a plant or other host cell.

12. A method for controlling flukes, which comprises administering to a host harbouring a fluke, or directly to the fluke, or to the situs of the fluke, a toxin from a wild-type Bacillus thuringiensis DNA which has been transformed into and expressed in a recombinant host.

13. A method according to claim 12, wherein the Bacillus thuringiensis is selected fromPS17, PS33F2, PS52A1, PS63B, PS69D1, PS80JJ1, PS158D5, PS167P, PS169E, PS177F1, PS177G, PS204G4, and PS204G6.

14. A method according to claim 12 or claim 13, wherein the DNA is according to claim 2.
